(19) Europäisches Patentamt
European Patent Office
Office européen des brevets

(11) Publication number: **0 186 120**
**B1**

(12) **EUROPEAN PATENT SPECIFICATION**

(45) Date of publication of patent specification: **23.11.88**

(51) Int. Cl.$^4$: **C 07 C 49/792,** C 07 C 121/64, C 07 C 147/06, C 07 C 149/32, A 01 N 35/06

(21) Application number: **85116167.9**

(22) Date of filing: **18.12.85**

(54) **Certain-2-(2'-alkylbenzoyl)-1,3-cyclohexanediones.**

(30) Priority: **20.12.84 US 683884**

(43) Date of publication of application:
**02.07.86 Bulletin 86/27**

(45) Publication of the grant of the patent:
**23.11.88 Bulletin 88/47**

(84) Designated Contracting States:
**AT BE CH DE FR GB IT LI NL SE**

(56) References cited:
**EP-A-0 017 195**
**EP-A-0 090 262**
**EP-A-0 135 191**
**EP-A-0 137 963**

(73) Proprietor: **STAUFFER CHEMICAL COMPANY**
**Westport Connecticut 06881 (US)**

(72) Inventor: **Carter, Charles Garvie**
**1240 Willard Street**
**San Francisco, Calif. 94117 (US)**

(74) Representative: **Kraus, Walter, Dr. et al**
**Patentanwälte Kraus, Weisert & Partner**
**Thomas-Wimmer-Ring 15**
**D-8000 München 22 (DE)**

**The file contains technical information submitted after the application was filed and not included in this specification**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

## Description

### Background of the Invention

Compounds having the stuctural formula

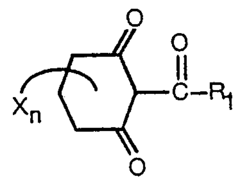

wherein X can be an alkyl, n can be 0, 1 or 2, and $R_1$ can be phenyl or substituted phenyl are described in Japanese Patent Application 84632—1974 as being intermediates for the preparation of herbicidal compounds of the formula

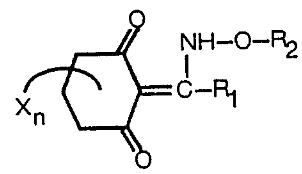

wherein $R_1$, X, and n are as defined above and $R_2$ is alkyl, alkenyl, or alkynyl. Specifically taught herbicidal compounds of this latter group are those where n is 2, X is 5,5-diphenyl, $R_2$ is allyl and $R_1$ is phenyl, 4-chlorophenyl or 4-methoxyphenyl.

The precursor intermediates for these three specifically taught compounds have no or almost no herbicidal activity.

European Patent Application No. 83 102 599.4 was published October 5, 1983 and relates to certain novel 2-(2-substituted benzoyl)-cyclohexane-1,3-diones as herbicides. The compounds have the following structural formula

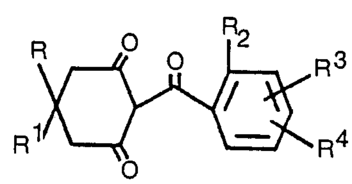

wherein R and $R^1$ are hydrogen or $C_1$—$C_4$ alkyl; $R^2$ is chlorine, bromine or iodine; $R^3$ is hydrogen or halogen; and $R^4$ is hydrogen, chlorine, bromine, iodine, $C_1$—$C_4$ alkyl, $C_1$—$C_4$ alkoxy, nitro or trifluoromethyl.

### Description of the Invention

This invention relates to 2-(2-alkylbenzoyl)-1,3-cyclohexanediones and their use as herbicides.

One embodiment of this invention is an herbicidal composition comprising an herbicidally active 2-benzoyl-1,3-cyclohexanedione and an inert carrier therefor wherein the 2-position of the benzoyl moiety is substituted with $C_1$—$C_4$ alkyl, preferably $C_1$—$C_2$ alkyl, optionally substituted with halogen, more preferably methyl or $CF_3$ and the 4-position preferably is substituted with an electron withdrawing group, such as halogen, cyano, $CF_3$ or nitro. The 4-, 5- and 6-positions of the 1,3-cyclohexanedione moiety can be substituted, preferably with the groups hereinafter recited. More preferably, the 1,3-cyclohexanedione moiety has no substitution or the 4- or 6-positions are substituted with one or two methyl groups. The 3-, 4- and 5-positions of the benzoyl moiety can be substituted, preferably with the groups hereinafter recited.

Also embodied within the scope of this invention are novel compounds having the following structural formula

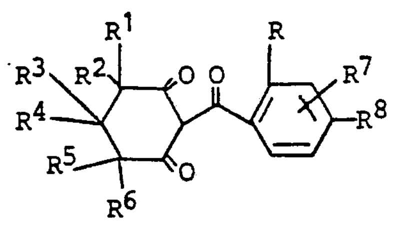

wherein

R is $C_1$—$C_4$ alkyl, preferably $C_1$—$C_2$ alkyl, optionally substituted with halogen, more preferably methyl and $CF_3$;

$R^1$ is hydrogen or $C_1$—$C_4$ alkyl, preferably $C_1$—$C_2$ alkyl, more preferably methyl, most preferably $R^1$ is hydrogen or methyl;

$R^2$ is hydrogen; $C_1$—$C_4$ alkyl, preferably $C_1$—$C_2$ alkyl, more preferably methyl or

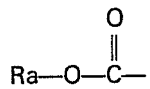

wherein $R^a$ is $C_1$—$C_4$ alkyl, most preferably $R^2$ is hydrogen or methyl; or

$R^1$ and $R^2$ together are alkylene having 3 to 6 carbon atoms;

$R^3$ is hydrogen or $C_1$—$C_4$ alkyl, preferably $C_1$—$C_2$ alkyl, more preferably methyl; most preferably $R^3$ is hydrogen or methyl;

$R^4$ is hydrogen or $C_1$—$C_4$ alkyl, preferably $C_1$—$C_2$ alkyl, more preferably methyl; most preferably $R^4$ is hydrogen or methyl;

$R^5$ is hydrogen or $C_1$—$C_4$ alkyl, preferably $C_1$—$C_2$ alkyl, more preferably methyl; most preferably $R^5$ is hydrogen or methyl;

$R^6$ is hydrogen or $C_1$—$C_4$ alkyl, preferably $C_1$—$C_2$ alkyl, more preferably methyl; most preferably $R^6$ is hydrogen;

$R^7$ and $R^8$ independently are (1) hydrogen; (2) halogen, preferably chlorine, fluorine or bromine; (3) $C_1$—$C_4$ alkly, preferably methyl; (4) $C_1$—$C_4$ alkoxy, preferably methoxy; (5) $OCF_3$; (6) cyano; (7) nitro; (8) $C_1$—$C_4$ haloalkyl, more preferably trifluoromethyl; (9) $R^bSO_n$- wherein n is the integer 0, 1 or 2, preferably 2; and $R^b$ is (a) $C_1$—$C_4$ alkyl, preferably methyl; (b) $C_1$—$C_4$ alkyl substituted with halogen or cyano, preferably chloromethyl, trifluoromethyl or cyanomethyl; (c) phenyl; or (d) benzyl) (10) —$NR^cR^d$ wherein $R^c$ and $R^d$ independently are hydrogen or $C_1$—$C_4$ alkyl; (11) $R^eC(O)$—wherein $R^e$ is $C_1$—$C_4$ alkyl or $C_1$—$C_4$ alkoxy; or (12) —$SO_2NR^cR^d$ wherein $R^c$ and $R^d$ are as defined, with the proviso that $R^7$ is not attached to the 6-position.

The term "$C_1$—$C_4$ alkyl" includes methyl, ethyl, n-propyl, isopropyl, n-butyl, sec-butyl, isobutyl and t-butyl. The term "halogen" includes chlorine, bromine, iodine and fluorine. The term "$C_1$—$C_4$ alkoxy" includes methoxy, ethoxy, n-propoxy, isopropoxy, n-butoxy, sec-butoxy, isobutoxy and t-butoxy. The term "haloalkyl" includes the eight alkyl groups with one or more hydrogens replaced by chloro, bromo, iodo or fluoro.

Preferably, $R^7$ is in the 3-position. More preferably $R^7$ is hydrogen and $R^8$ is hydrogen, chlorine, bromine, fluorine, $CF_3$, or $R^bSO_2$ wherein $R^b$ is $C_1$—$C_4$ alkyl, preferably methyl.

Salts of the above-described compounds (as defined hereinafter) are also the subject of the instant invention.

The compounds of this invention can have the following four structural formulae because of tautomerism

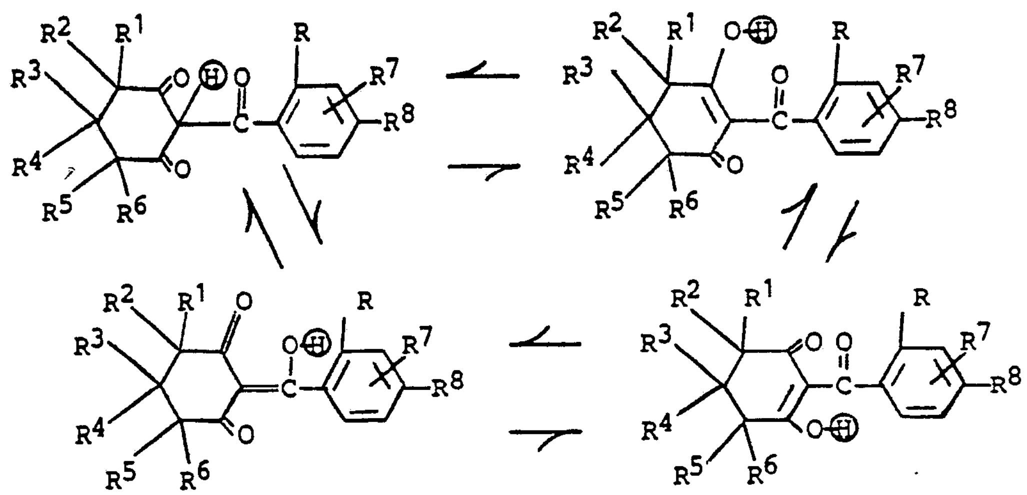

wherein R, $R^1$, $R^2$, $R^3$, $R^4$, $R^5$, $R^6$, $R^7$ and $R^8$ are as defined above.

The circled proton on each of the four tautomers is reasonably labile. These protons are acidic and can be removed by any base to give a salt having an anion of the following four resonance forms:

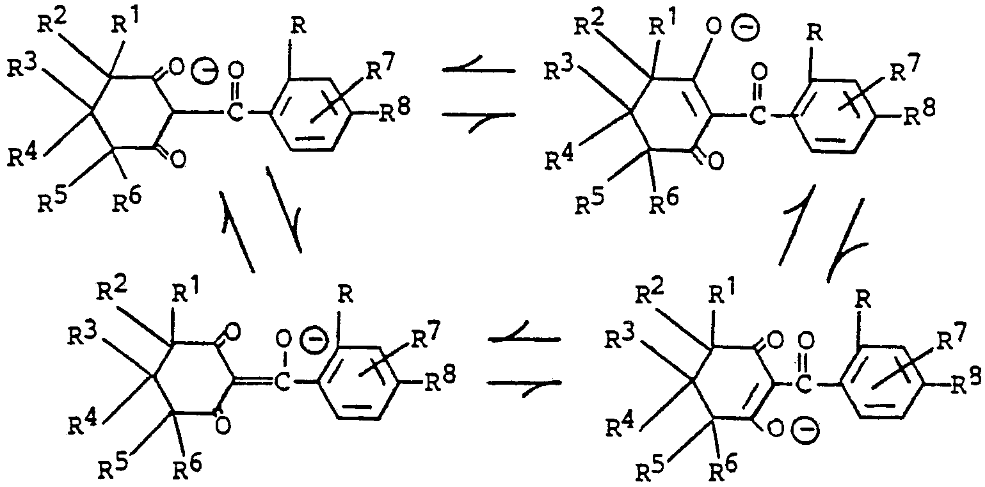

wherein R, $R^1$, $R^2$, $R^3$, $R^4$, $R^5$, $R^6$, $R^7$ and $R^8$ are as defined above.

Examples of cations of these bases are inorganic cations such as alkali metals e.g. lithium, sodium, and potassium organic cations such as substituted ammonium, sulfonium or phosphonium wherein the substituent is an aliphatic or aromatic group.

The compounds of this invention and their salts are active herbicides of a general type. That is, they are herbicidally effective against a wide range of plant species. The method of controlling undesirable vegetation of the present invention comprises applying an herbicidally effective amount of the above-described compounds to the area where control is desired,

The compounds of the present invention can be prepared by the following two-step general method.

The process proceeds via the production of an enol ester intermediate as shown in reaction (1). The final product is obtained by rearrangement of the enol ester as shown in reaction (2). The two reactions may be conducted as separate steps by isolation and recovery of the enol ester using conventional techniques prior to conducting step (2), or by addition of a cyanide source to the reaction medium after the formation of the enol ester, or in one step by inclusion of the cyanide source at the start of reaction (1).

1)

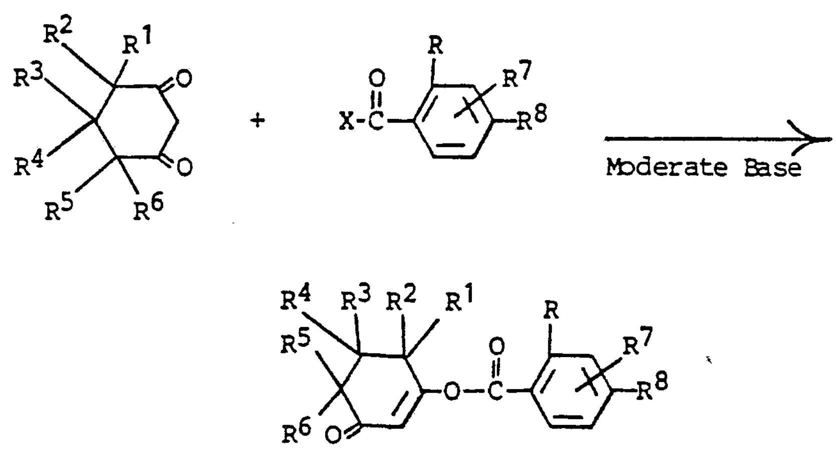


wherein R through $R^8$ are as defined and X is halogen, preferably, chlorine, $C_1$—$C_4$ alkyl—C(O)—O—, $C_1$—$C_4$ alkoxy—C(O)—O— or

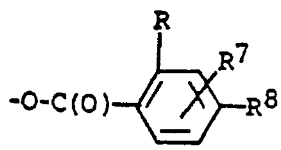

wherein R, $R^7$ and $R^8$ in this portion of the molecule are identical with those in the reactant shown above and the moderate base is as defined, preferably tri-$C_1$—$C_6$ alkylamine, pyridine, alkali metal carbonate or alkali metal phosphate.

Generally, in step (1) mole amounts of the dione and substituted benzoyl reactant are used, along with a mole amount or excess of the base. The two reactants are combined in an organic solvent such as

methylene chloride, toluene, ethyl acetate or dimethylformamide. The base or benzoyl reactant preferably are added to the reaction mixture with cooling. The mixture is stirred at 0°C—50°C until the reaction is substantially complete.

The reaction product is worked up by conventional techniques.

2)

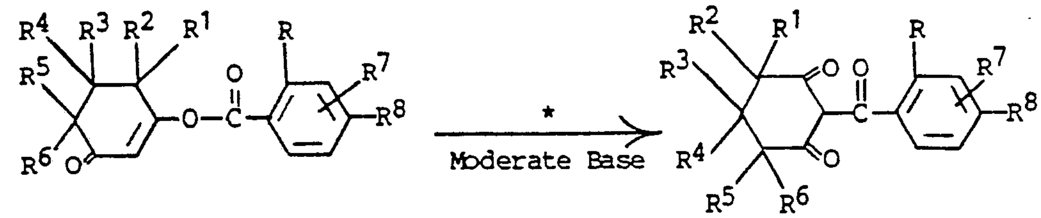


* = Cyanide source.

Moderate base = as defined herein.

wherein R through $R^8$ are as defined.

Generally, in step (2) a mole of the enol ester intermediate is reacted with 1 to 4 moles of the base, preferably about 2 moles of moderate base and from 0.01 mole to about 0.5 mole or higher, preferably around 0.1 mole of the cyanide source (e.g., potassium cyanide or acetone cyanohydrin). The mixture is stirred in a reaction pot until the rearrangement is substantially complete at a temperature below 80°C, preferably about 20°C to about 40°C, and the desired product is recovered by conventional techniques.

The term "cyanide source" refers to a substance or substances which under the rearrangement conditions consists of or generates hydrogen cyanide and/or cyanide anion.

The process is conducted in the presence of a catalytic amount of a source of cyanide anion and/or hydrogen cyanide, together with a molar excess, with respect to the enol ester, of a moderate base.

Preferred cyanide sources are alkali metal cyanides such as sodium potassium cyanide; cyanohydrins of methyl alkyl ketones having from 1—4 carbon atoms in the alkyl groups, such as acetone or methyl isobutyl ketone cyanohydrins; cyanohydrins of benzaldehyde or of $C_2$—$C_5$ aliphatic aldehydes such as acetaldehyde, propionaldehyde, etc., cyanohydrins; zinc cyanide; tri(lower alkyl) silyl cyanides, notably trimethyl silyl cyanide; and hydrogen cyanide itself. Hydrogen cyanide is considered most advantageous as it produces relatively rapid reaction and is inexpensive. Among cyanohydrins the preferred cyanide source is acetone cyanohydrin.

The cyanide source is used in an amount up to about 50 mole percent based on the enol ester. It may be used in as little as about 1 mole percent to produce an acceptable rate of reaction at about 40°C on a small scale. Larger scale reactions give more reproducible results with slightly higher catalyst levels of about 2 mole percent. Generally about 1—10 mole% of the cyanide source is preferred.

The process is conducted with a molar excess, with respect to the enol ester, of a moderate base. By the term "moderate base" is meant a substance which acts as a base yet whose strength or activity as a base lies between that of strong bases such as hydroxides (which could cause hydrolysis of the enol ester) and that of weak bases such as bicarbonates (which would not function effectively). Moderate bases suitable for use in this embodiment include both organic bases such as tertiary amines and inorganic bases such as alkali metal carbonates and phosphates. Suitable tertiary amines include trialkylamines such as triethylamine, trialkanolamines such as triethanolamine, and pyridine. Suitable inorganic bases include potassium carbonate and trisodium phosphate.

The base is used in an amount of from about 1 to about 4 moles per mole of enol ester, preferably about 2 moles per mole.

When the cyanide source is an alkali metal cyanide, particularly potassium cyanide, a phase transfer catalyst may be included in the reaction. Particularly suitable phase transfer catalysts are the Crown ethers.

A number of different solvents may be usable in this process, depending on the nature of the acid chloride or the acylated product. A preferred solvent for this reaction is 1,2-dichloroethane. Other solvents which may be employed, depending on the reactants or products include toluene, acetonitrile, methylene chloride, ethyl acetate, dimethylformamide, and methyl isobutyl ketone (MIBK).

In general, depending on the nature of the reactants and the cyanide source, the rearrangement may be conducted at temperatures up to about 50°C.

The above described substituted benzoyl chlorides can be prepared from the corresponding substituted benzoic acids according to the teaching of *Reagants for Organic Synthesis, Vol. I,* L. F. Fieser and M. Fieser, pp 767—769 (1967).

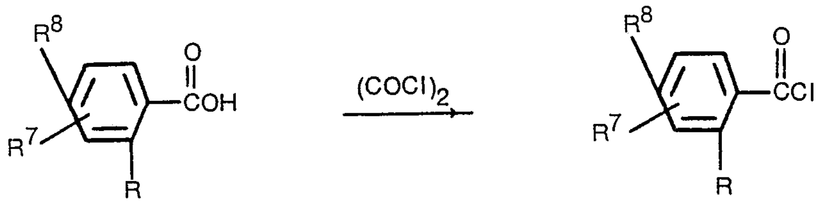


wherein R, $R^7$ and $R^8$ are as previously defined.

The substituted benzoic acids can be prepared by a wide variety of general methods according to the teaching of *The Chemistry of Carboxylic Acids and Esters,* S. Patai, editor, J. Wiley and Sons, New York, N.Y. (1969) and *Survey of Organic Synthesis,* C. A. Buehler and D. F. Pearson, J. Wiley and Sons, (1970).

The following are four representative examples of the methods described therein.

a) 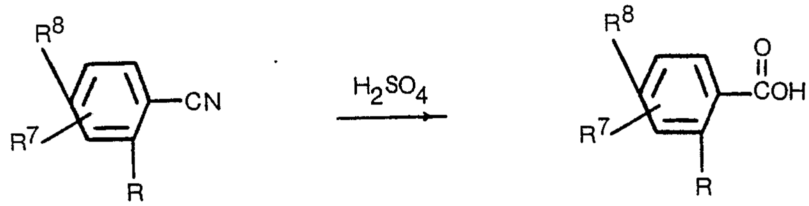


wherein R, $R^7$ and $R^8$ are as previously defined.

In reaction (a) the substituted benzonitrile is heated to reflux in aqueous sulfuric acid for several hours. The mixture is cooled and the reaction product is isolated by conventional techniques.

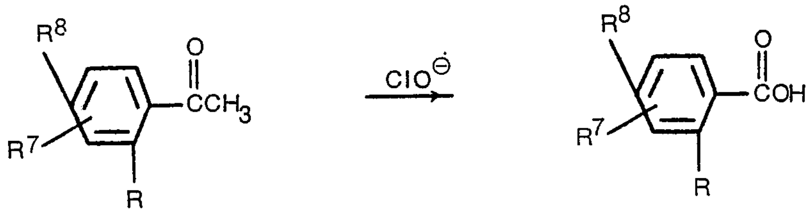


wherein R, $R^7$ and $R^8$ are as previously defined.

In reaction (b) the substituted acetophenone is heated to reflux for several hours in an aqueous hypochlorite solution. The mixture is cooled and the reaction product is isolated by conventional techniques.

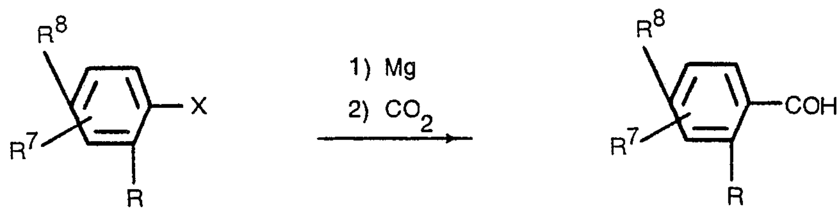


wherein R, $R^7$ and $R^8$ are as defined and X is chlorine, bromine or iodine.

The substituted aromatic halide is allowed to react with magnesium in a solvent such as ether. The solution is then poured over crushed dry ice and the benzoic acid is isolated by conventional techniques.

The following example teaches the synthesis of a representative compound of this invention.

Example 1

2-(4′-Bromo-2′-trifluoromethylbenzoyl)-4,4,6-trimethyl-1,3-cyclohexanedione

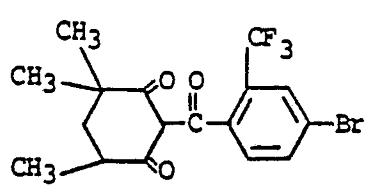

4-Bromo-2-trifluoromethylbenzoyl chloride (4.3 g, 15 mmol) and 4,4,6-trimethyl-1,3-cyclohexanedione (2.3 g, 15 mmol) were dissolved in 100 ml methylene chloride. The solution was cooled with an ice bath and triethylamine (2.1 ml, 15 mmol) in 10 ml methylene chloride was added dropwise. The ice bath was then

removed and the resulting solution stirred for 30 minutes at room temperature. The solution was washed with 2N hydrochloric acid (2N HCl), 5% potassium carbonate solution (5% $K_2CO_3$) and saturated sodium chloride solution (brine), dried over anhydrous magnesium sulfate ($MgSO_4$) and concentrated under vacuum. The residue (5.1 g) was dissolved in 20 ml acetonitrile. Triethylamine (3.5 ml, 25 mmol) and 0.4 ml acetone cyanohydrin were added and the solution stirred for two hours hours at room temperature while protected by a drying tube (calcium sulfate). After dilution with ether, the solution was washed with 2N HCl and extracted with 5% $K_2CO_3$. The aqueous extract was acidified with concentrated hydrochloric acid and extracted with ether. The ether was washed with brine, dried ($MgSO_4$) and concentrated under vacuum. The resulting oil was purified on a silica gel column (80:20:1 hexane:ethyl acetate:acetic acid — eluent), yielding 1.5 g of a viscous oil which was identified as the desired compound by nuclear magnetic resonance spectroscopy, infrared spectroscopy and mass spectroscopy.

The following is a table of certain selected compounds that are preparable according to the procedure described hereto. Compound numbers are assigned to each compound and are used throughout the remainder of the application.

TABLE I

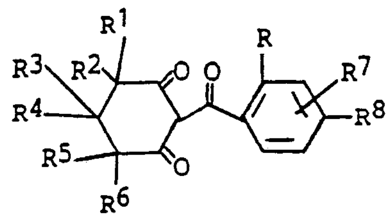

| Comp. No. | R | $R^1$ | $R^2$ | $R^3$ | $R^4$ | $R^5$ | $R^6$ | $R^7$ | $R^8$ | $n_D^{30}$ or m.p. |
|---|---|---|---|---|---|---|---|---|---|---|
| 1 | $CH_3$ | H | H | H | H | H | H | H | H | 35–42°C |
| 2 | $CH_3$ | $CH_3$ | $CH_3$ | H | H | H | H | H | H | 47–53°C |
| 3 | $CH_3$ | $CH_3$ | $CH_3$ | H | H | H | H | H | Br | oil |
| 4 | $CH_3$ | $CH_3$ | $CH_3$ | H | H | H | H | H | CN | oil |
| 5 | $CH_3$ | $CH_3$ | $CH_3$ | H | H | H | H | 3-$NO_2$ | H | oil |
| 6 | $CH_3$ | $CH_3$ | $CH_3$ | H | H | H | H | 5-Cl | H | oil |
| 7 | $CH_3$ | $CH_3$ | $CH_3$ | H | H | H | H | H | $CH_3SO_2$- | oil |
| 8 | $CH_3$ | $CH_3$ | $CH_3$ | H | H | $CH_3$ | H | H | $CH_3SO_2$- | oil |
| 9 | $CH_3$ | $CH_3$ | $CH_3$ | H | H | H | H | 3-Cl | H | oil |
| 10 | $CH_3$ | $CH_3$ | $CH_3$ | H | H | $CH_3$ | H | H | $C_2H_5SO_2$- | oil |
| 11 | $CH_3$ | $CH_3$ | $CH_3$ | H | H | H | H | H | $C_2H_5SO_2$- | oil |
| 12 | $CH_3$ | $CH_3$ | $CH_3$ | H | H | H | H | H | n-$C_3H_7SO_2$- | oil |
| 13 | $CH_3$ | H | H | H | H | H | H | H | $CH_3SO_2$- | oil |
| 14 | $CH_3$ | H | H | H | H | H | H | H | n-$C_3H_7SO_2$- | oil |
| 15 | $CH_3$ | $CH_3$ | $CH_3$ | H | H | H | H | H | $CH_3S$- | oil |
| 16 | $C_2H_5$ | $CH_3$ | $CH_3$ | H | H | H | H | H | Br | oil |
| 17 | $CH_3$ | H | H | H | H | H | H | H | CN | oil |
| 18 | $CH_3$ | $CH_3$ | $CH_3$ | H | H | H | H | H | F | oil |
| 19 | $CH_3$ | H | H | H | H | H | H | H | $C_2H_5$-$SO_2$- | oil |
| 20 | $CH_3$ | H | H | H | H | H | H | 3-Cl | H | 65–67°C |
| 21 | $CH_3$ | H | H | H | H | H | H | 3-I | H | oil |
| 22 | $CH_3$ | H | H | H | H | H | H | 3-$NO_2$ | H | oil |
| 23 | $CH_3$ | H | H | H | H | H | H | 3-CN | H | 96–101°C |
| 24 | $CF_3$ | $CH_3$ | $CH_3$ | H | H | H | H | H | H | oil |

TABLE I

(continued)

| Comp. No. | R | $R^1$ | $R^2$ | $R^3$ | $R^4$ | $R^5$ | $R^6$ | $R^7$ | $R^8$ | $n_D^{30}$ or m.p. |
|---|---|---|---|---|---|---|---|---|---|---|
| 25 | $CF_3$ | H | H | H | H | H | H | H | H | oil |
| 26 | $CF_3$ | $CH_3$ | $CH_3$ | H | H | H | H | H | Br | oil |
| 27 | $CF_3$ | H | H | H | H | H | H | H | Cl | 82-88°C |
| 28 | $CF_3$ | $CH_3$ | $CH_3$ | H | H | H | H | H | Cl | oil |
| 29 | $CF_3$ | H | H | H | H | H | H | H | $C_2H_5S-$ | oil |
| 30 | $CF_3$ | $CH_3$ | $CH_3$ | H | H | H | H | H | $C_2H_5SO_2-$ | oil |
| 31 | $CF_3$ | H | H | H | H | H | H | H | CN | oil |
| 32 | $CF_3$ | $CH_3$ | $CH_3$ | H | H | H | H | H | CN | oil |
| 33a | $CF_3$ | $CH_3$ | $CH_3$ | H | H | $CH_3$ | H | H | Br | oil |
| 34 | $CH_3$ | H | H | H | H | H | H | H | $CH_3$ | |
| 35 | $CH_3$ | $CH_3$ | $CH_3$ | H | H | H | H | 3-Cl | $C_2H_5SO_2$ | 115-117°C |
| 36 | $CH_3$ | H | H | H | H | H | H | 3-Cl | $C_2H_5SO_2$ | oil |
| 37 | $CH_3$ | b) | b) | H | H | H | H | 3-$CF_3$ | H | oil |
| 38 | $CH_3$ | c) | H | i-$C_3H_7$ | H | H | H | 3-$NO_2$ | H | 88-108°C |
| 39 | $CF_3$ | $CH_3$ | $CH_3$ | H | H | $CH_3$ | H | H | $CH_3S$ | oil |
| 40 | $CF_3$ | $CH_3$ | $CH_3$ | H | H | H | H | H | $CH_3S$ | oil |
| 41 | $CF_3$ | $CH_3$ | $CH_3$ | H | H | $CH_3$ | H | H | $CH_3SO_2-$ | oil |
| 42 | $CH_3$ | $CH_3$ | $CH_3$ | H | H | H | H | H | $CF_3$ | oil |
| 43 | $CH_3$ | H | H | H | H | H | H | H | $CF_3$ | 114-120°C |
| 44 | $CH_3$ | H | H | H | H | H | H | 3-Cl | Cl | oil |
| 45 | $CH_3$ | $CH_3$ | $CH_3$ | H | H | H | H | 3-Cl | Cl | oil |
| 46 | $CH_3$ | $CH_3$ | $CH_3$ | H | H | H | H | 3-$CF_3$ | H | oil |
| 47 | $CF_3$ | H | H | H | H | H | H | H | $CH_3S$ | oil |
| 48 | $CF_3$ | $CH_3$ | $CH_3$ | H | H | $CH_3$ | H | H | $CF_3$ | oil |
| 49 | $CF_3$ | H | H | H | H | H | H | H | $CF_3$ | oil |
| 50 | $CF_3$ | $CH_3$ | $CH_3$ | H | H | H | H | H | $CF_3$ | oil |
| 51 | $CH_3$ | H | H | $CH_3$ | H | H | H | H | $CH_3SO_2$ | oil |
| 52 | $CF_3$ | $CH_3$ | $CH_3$ | H | H | $CH_3$ | H | H | $C_2H_5SO_2$ | oil |
| 53 | $CH_3$ | H | H | H | H | H | H | H | Br | 94-98°C |
| 54 | $CH_3$ | H | H | H | H | H | H | d) | H | oil |

a) Prepared in Example 1.

b) $-(CH_2)_5-$

c) $C_2H_5OC(O)-$

d) 3-$N(CH_3)COCH_3$

Herbicidal Screening Tests

As previously mentioned, the herein described compounds produced in the above-described manner are phytotoxic compounds which are useful and valuable in controlling various plant species. Selected compounds of this invention were tested as herbicides in the following manner.

Pre-emergence herbicide test.

On the day preceding treatment, seeds of eight different weed species are planted in loamy sand soil in individual rows using one species per row across the width of a flat. The weeds used are green foxtail (FT) *(Setaria viridis)*, watergrass (WG) *(Echinochloa crusgalli)*, wild oat (WO) *(Avena fatua)*, annual morningglory (AMG) *(Ipomoea lacunosa)*, velvetleaf (VL) *(Abutilon theophrasti)*, Indian mustard (MD) *(Brassica juncea)*, curly dock (CD) *(Rumex crispus)*, and yellow nutsedge (YNG) *(Cyperus esculentus)*. Ample seeds are planted to give about 20 to 40 seedlings per row, after emergence, depending upon the size of the plants.

Using an analytical balance, 600 milligrams (mg) of the compound to be tested are weighed out on a piece of glassine weighing paper. The paper and compound are placed in a 60 milliliter (ml) wide-mouth clear bottle and dissolved in 45 ml of acetone or substituted solvent. Eighteen ml of this solution are transferred to a 60 ml wide-mouth clear bottle and diluted with 22 ml of a water and acetone mixture (19:1) containing enough polyoxyethylene sorbitan monolaurate emulsifier to give a final solution of 0.5% (v/v). The solution is then sprayed on a seeded flat on a linear spray table calibrated to deliver 80 gallons per acre (748 L/ha). The application rate is 4 lb/acre (4.48 Kg/ha).

After treatment, the flats are placed in the greenhouse at a temperature of 70 to 80°F and watered by sprinkling. Two weeks after treatment, the degree of injury or control is determined by comparison with untreated check plants of the same age. The injury rating from 0 to 100% is recorded for each species as percent control with 0% representing no injury and 100% representing complete control.

The results of the tests are shown in the following Table II.

TABLE II

Pre-Emergence Herbicidal Activity
Application Rate -- 4.48 kg/ha

| Cmpd. No. | FT | WG | WO | AMG | VL | MD | CD | YNG |
|---|---|---|---|---|---|---|---|---|
| 1 | 40 | 20 | 0 | 0 | 0 | 0 | 0 | 100 |
| 2 | 60 | 70 | 0 | 0 | 0 | 0 | 90 | 100 |
| 3 | 100 | 100 | 50 | 50 | 100 | 100 | 85 | 90 |
| 4 | 100 | 100 | 90 | 30 | 100 | 85 | 95 | 95 |
| 5 | 100 | 100 | 80 | 10 | 100 | 100 | 100 | 95 |
| 6 | 20 | 35 | 25 | 15 | 90 | 85 | 40 | 85 |
| 7 | 100 | 100 | 90 | 100 | 100 | 100 | 100 | 95 |
| 8 | 100 | 100 | 90 | 100 | 100 | 100 | 100 | 95 |
| 9 | 100 | 100 | 0 | 0 | 100 | 80 | 100 | 90 |
| 10 | 100 | 100 | 100 | 100 | 100 | 100 | 100 | 95 |
| 11 | 100 | 100 | 70 | 100 | 100 | 100 | 97 | 95 |
| 12 | 100 | 100 | 60 | 100 | 100 | 100 | 100 | 95 |
| 13 | 100 | 100 | 60 | 100 | 100 | 100 | 100 | 95 |
| 14 | 80 | 100 | 50 | 80 | 100 | 100 | 90 | 90 |
| 15 | 100 | 100 | 80 | 100 | 100 | 100 | 100 | - |
| 16 | 50 | 75 | 0 | 25 | 100 | 100 | 95 | 60 |
| 17 | 100 | 100 | 40 | 100 | 100 | 100 | 100 | 85 |
| 18 | 100 | 100 | 0 | 20 | 100 | 100 | 80 | 70 |
| 20 | 70 | 75 | 0 | 25 | 100 | 95 | 100 | 60 |
| 21 | 50 | 60 | 0 | 0 | 100 | 80 | 80 | 60 |
| 22 | 100 | 95 | 35 | 25 | 100 | 100 | 90 | 50 |
| 23 | 95 | 100 | 40 | 20 | 100 | 100 | 90 | 50 |
| 24 | 100 | 100 | 90 | 0 | 45 | 85 | 80 | 90 |
| 25 | 100 | 100 | 25 | 60 | 100 | 100 | 100 | 75 |
| 34 | 35 | 40 | 10 | 0 | 60 | 25 | 0 | 70 |
| 37 | 50 | 60 | 0 | 0 | 60 | 0 | 50 | 0 |
| 38 | 35 | 40 | 0 | 0 | 0 | 0 | 0 | 0 |
| 52 | 85 | 100 | 30 | 95 | 100 | 100 | - | 80 |
| 53 | 100 | 100 | 0 | 85 | 100 | 100 | - | 80 |
| 54 | 100 | 100 | 90 | 25 | 100 | 100 | - | 80 |

A blank (-) indicates that the weed was not tested.

Post-Emergence Herbicide Test

This test is conducted in an identical manner to the testing procedure for the pre-emergence herbicide test, except the seeds of the eight different weed species are planted 10—12 days before treatment. Also, watering of the treated flats is confined to the soil surface and not to the foliage of the sprouted plants.

The results of the post-emergence herbicide test are reported in Table III.

TABLE III

Post-Emergence Herbicidal Activity
Application Rate -- 4.48 kg/ha

| Cmpd. No. | FT | WG | WO | AMG | VL | MD | CD | YNG |
|---|---|---|---|---|---|---|---|---|
| 1 | 60 | 40 | 20 | 30 | 40 | 40 | 50 | 60 |
| 2 | 50 | 40 | 10 | 20 | 20 | 20 | 40 | 60 |
| 3 | 100 | 100 | 85 | 100 | 100 | 100 | 100 | 75 |
| 4 | 100 | 100 | 95 | 85 | 100 | 95 | 100 | 60 |
| 5 | 95 | 95 | 100 | 65 | 90 | 25 | 70 | 45 |
| 6 | 40 | 30 | 0 | 20 | 90 | 25 | 20 | 70 |
| 7 | 100 | 100 | 100 | 100 | 100 | 100 | 100 | 90 |
| 8 | 100 | 95 | 100 | 100 | 100 | 100 | 100 | 70 |
| 9 | 65 | 65 | 0 | 20 | 80 | 65 | 90 | 80 |
| 10 | 100 | 100 | 100 | 100 | 100 | 100 | 100 | 70 |
| 11 | 85 | 90 | 90 | 85 | 90 | 80 | 30 | 60 |
| 12 | 100 | 90 | 65 | 80 | 100 | 100 | 100 | 50 |
| 13 | 100 | 95 | 100 | 100 | 100 | 100 | 90 | - |
| 14 | 100 | 100 | 100 | 100 | 100 | 100 | 100 | 50 |
| 15 | 25 | 35 | 15 | 30 | 80 | 25 | 20 | 0 |
| 16 | 100 | 85 | 70 | 75 | 90 | 90 | 50 | - |
| 17 | 100 | 100 | 100 | 100 | 100 | 100 | 100 | - |
| 18 | 90 | 85 | 85 | 85 | 90 | 95 | 70 | 40 |
| 20 | 40 | 60 | 10 | 60 | 100 | 100 | 100 | 50 |
| 21 | 35 | 60 | 10 | 60 | 100 | 100 | 80 | 60 |
| 22 | 95 | 95 | 35 | 100 | 100 | 100 | 90 | 50 |
| 23 | 100 | 100 | 40 | 100 | 100 | 100 | 90 | 50 |
| 24 | 100 | 75 | 100 | 60 | - | 100 | 100 | 60 |
| 25 | 95 | 95 | 90 | 95 | 100 | 100 | 95 | 35 |
| 34 | 50 | 40 | 0 | 35 | 50 | 70 | 30 | 50 |
| 37 | 20 | 60 | 0 | 30 | 30 | 50 | 50 | 50 |
| 38 | 25 | 50 | 0 | 25 | 25 | 50 | 20 | 20 |
| 52 | 0 | 60 | 50 | 50 | 10 | 50 | - | 20 |
| 53 | 0 | 50 | 0 | 50 | 50 | 50 | - | 30 |
| 54 | 90 | 75 | 60 | 50 | 50 | 80 | - | 80 |

A blank (-) indicates the weed was not tested.

Pre-Emergence Multi-Weed Herbicide Test

Several compounds were evaluated at an application rate of 1 or ½ lb/acre (1.12 or 0.56 kg/ha) for pre-emergence activity against a larger number of weed species.

Pre-Emergence Multi-Weed Herbicide Test

Several compounds were evaluated at an application rate of 1 or ½ lb/acre (1.12 or 0.56 kg/ha) for pre-emergence activity against a larger number of weed species.

The process was generally similar to the pre-emergence herbicide test described above except that only 150 or 75 milligrams of test compound were weighed out and the application rate was 40 gallons per acre.

Redroot pigweed (PG) and curly dock (CD) were eliminated in this test and the following weed species were added:

| | | | |
|---|---|---|---|
| Grasses: | downy brome | *Bromus tectorum* | (DB) |
| | annual ryegrass | *lolium multiflorum* | (ARG) |
| | Johnsongrass | *Sorghum halepense* | (JG) |
| | broadleaf signalgrass | *Brachiaria platyphylla* | (BSG) |
| | hemp sesbania | *Sesbania exaltata* | (SESB) |
| | sicklepod | *Cassia obtusifolia* | (SP) |
| | cocklebur | *Xanthium sp.* | (CB) |

The results of the test are shown in Tables IV and V.

TABLE IV

Pre-Emergence Multi-weed Herbicide Test

Application Rate - 1.12 kg/ha

| Cmpd. No. | DB | FT | ARG | WG | JG | WO | BSG | AMG | SESB | VL | SP | MD | YNG | CB |
|---|---|---|---|---|---|---|---|---|---|---|---|---|---|---|
| 19 | 40 | 35 | 60 | 100 | 90 | 30 | 90 | 100 | 100 | - | 20 | 100 | 90 | - |
| 26 | 25 | 70 | 45 | 95 | 60 | 25 | 85 | 35 | 75 | - | 20 | 100 | 95 | 90 |
| 27 | 45 | 65 | 40 | 100 | 60 | 25 | 80 | 100 | 85 | 100 | 40 | 100 | 95 | 35 |
| 28 | 80 | 100 | 90 | 100 | 100 | 90 | 100 | 70 | 90 | 100 | 30 | 100 | 95 | 35 |
| 29 | 90 | 45 | 75 | 100 | 85 | 40 | 90 | 95 | 90 | 100 | 40 | 100 | 95 | - |
| 30 | 90 | 95 | 80 | 100 | 90 | 75 | 100 | 100 | 80 | 100 | 60 | 100 | 95 | - |
| 31 | 60 | 65 | 75 | 100 | 100 | 55 | 95 | 100 | 100 | 100 | 100 | 100 | 95 | - |
| 32 | 90 | 100 | 90 | 100 | 100 | 65 | 85 | 100 | 90 | 100 | 95 | 100 | 85 | - |
| 33 | 100 | 100 | 95 | 100 | 100 | 95 | 100 | 100 | 100 | 100 | 25 | 100 | 90 | - |
| 35 | 90 | 100 | 100 | 100 | - | 90 | 90 | 100 | 90 | 100 | 10 | 100 | 75 | - |
| 36 | 90 | 35 | 85 | 100 | - | 15 | 80 | 95 | 90 | 100 | 50 | 100 | 95 | - |
| 39 | 100 | 100 | 100 | 100 | 100 | 95 | 100 | 100 | 100 | 100 | 100 | 100 | 98 | - |
| 40 | 100 | 100 | 80 | 100 | 100 | 70 | 100 | 100 | 100 | 100 | 90 | 100 | 95 | - |
| 41 | 100 | 100 | 100 | 100 | 100 | 100 | 100 | 100 | 100 | 100 | 60 | 100 | 98 | - |
| 42 | 60 | 100 | 85 | 100 | 100 | 70 | 95 | 100 | 95 | 100 | 10 | 100 | 95 | 50 |
| 43 | 85 | 100 | 25 | 100 | 60 | 25 | 98 | 100 | 100 | 100 | 100 | 100 | 90 | 100 |
| 44 | 0 | 80 | 10 | 85 | 0 | 0 | 75 | 0 | 30 | 100 | 0 | 100 | 0 | 10 |
| 45 | 25 | 100 | 40 | 100 | 60 | 0 | 65 | 60 | 60 | 100 | 50 | 100 | 70 | 0 |
| 50 | - | 100 | 50 | 100 | 35 | 35 | 75 | | 100 | 100 | - | | | |

(-) = Not tested.

TABLE V

Pre-Emergence Multi-weed Herbicide Test

Application Rate - 0.56 kg/ha

| Cmpd. No. | DB | FT | ARG | WG | JG | WO | BSG | AMG | SESB | VL | SP | MD | YNG | CB |
|---|---|---|---|---|---|---|---|---|---|---|---|---|---|---|
| 46 | 0 | 65 | 35 | 75 | 65 | 0 | 0 | 0 | 35 | 100 | 25 | 90 | 80 | 0 |
| 47 | - | 60 | 35 | 100 | 100 | 0 | 90 | 100 | 100 | 100 | 70 | - | 100 | 75 |
| 48 | - | 75 | 20 | 90 | 60 | 0 | 15 | 70 | 35 | 90 | 0 | - | 20 | 40 |
| 49 | - | 60 | 10 | 100 | 60 | 0 | 50 | 100 | 95 | 100 | 40 | - | 90 | 100 |
| 51 | - | 40 | 10 | 95 | 40 | 10 | 50 | 65 | 100 | 100 | 0 | - | 95 | 65 |

(-) - Not tested.

Post-Emergence Multi-Weed Herbicide Test

This test is conducted in an identical manner to the testing procedure for the post-emergence herbicide test, except the seeds of the eight weed species used in the pre-emergence multi-weed herbicide test were used and the seeds were planted 10—12 days before treatment. Also, watering of the untreated flats is confined to the soil surface and not to the foliage of the sprouted plants.

The results of the post-emergence multi-weed herbicide test are reported in Table VI and VII

TABLE VI

Post-Emergence Multi-Weed Herbicidal Activity
Application Rate — 2.24 kg/ha

| Cmpd. No. | DB | FT | ARG | WG | JG | WO | BSG | AMG | SESB | VL | SP | MD | YNG | CB |
|---|---|---|---|---|---|---|---|---|---|---|---|---|---|---|
| 19 | 100 | 90 | 65 | 100 | 100 | 98 | 100 | 100 | 100 | 100 | 60 | 100 | 70 | 100 |
| 26 | 60 | 75 | 60 | 95 | 45 | 50 | 90 | 50 | 90 | 100 | 40 | 90 | 60 | - |
| 27 | 80 | 70 | 65 | 95 | 100 | 90 | 100 | 100 | 100 | 100 | 85 | 100 | 75 | - |
| 28 | 80 | 100 | 75 | 90 | 85 | 80 | 100 | 80 | 80 | 100 | 30 | 100 | 85 | 85 |
| 29 | 85 | 70 | 55 | 90 | 98 | 90 | 100 | 100 | 100 | 100 | 35 | 100 | 60 | 100 |
| 30 | 90 | 80 | 40 | 90 | 85 | 80 | 80 | 70 | 100 | 100 | 20 | 100 | 40 | - |
| 31 | 100 | 80 | 20 | 100 | 100 | 100 | 100 | 100 | 100 | 100 | 100 | 100 | 85 | - |
| 32 | 70 | 90 | 60 | 100 | 100 | 98 | 100 | 100 | 100 | 100 | 100 | 100 | 60 | - |
| 33 | 95 | 100 | 80 | 100 | 90 | 100 | 100 | 100 | 100 | 100 | 70 | 100 | 40 | - |
| 35 | 70 | 90 | 40 | 100 | - | 100 | 100 | 100 | 100 | 100 | 98 | 100 | 30 | - |
| 36 | 85 | 100 | 35 | 100 | - | 20 | 100 | 100 | 100 | 100 | 100 | 100 | 15 | - |
| 39 | 100 | 100 | 85 | 100 | 100 | 100 | 100 | 100 | 100 | 100 | 100 | 100 | 90 | 100 |
| 40 | 100 | 100 | 30 | 100 | 100 | 100 | 100 | 100 | 100 | 100 | 90 | 100 | 75 | - |
| 41 | 100 | 100 | 35 | 90 | 70 | 100 | 90 | 100 | 100 | 100 | 50 | 100 | 70 | - |
| 42 | 45 | 100 | 50 | 100 | 70 | 55 | 80 | 100 | 100 | 100 | 70 | 100 | 75 | 100 |
| 43 | 25 | 80 | 15 | 98 | 35 | 0 | 20 | 100 | 100 | 100 | 100 | 100 | 60 | 95 |
| 44 | 0 | 15 | 0 | 65 | 0 | 0 | 0 | 70 | 100 | 100 | 40 | 100 | 0 | 100 |
| 45 | 0 | 75 | 35 | 70 | 35 | 0 | 60 | 50 | 85 | 100 | 50 | 100 | 75 | 70 |
| 50 | - | 40 | 0 | 75 | 45 | 40 | 60 | 100 | 100 | 95 | 30 | - | 60 | 100 |

(-) = Not tested.

TABLE VII

Post-Emergence Multi-Weed Herbicidal Activity
Application Rate — 0.56 kg/ha

| Cmpd. No. | DB | FT | ARG | WG | JG | WO | BSG | AMG | SESB | VL | SP | MD | YNG | CB |
|---|---|---|---|---|---|---|---|---|---|---|---|---|---|---|
| 46 | 25 | 35 | 20 | 50 | 35 | 35 | 0 | 20 | 75 | 100 | 35 | 95 | - | 50 |
| 47 | - | 75 | 25 | 90 | 40 | 25 | 80 | 100 | 100 | 100 | 80 | - | 75 | 100 |
| 48 | - | 50 | 35 | 80 | 35 | 40 | 0 | 75 | 100 | 85 | 15 | - | 50 | 75 |
| 49 | - | 35 | 0 | 75 | 30 | 0 | 20 | 100 | 100 | 100 | 80 | - | 25 | 85 |
| 51 | - | 35 | 0 | 80 | 35 | 20 | 70 | 50 | 95 | 95 | 25 | - | 65 | 50 |

(-) = Not Tested.

The compounds of the present invention are useful as herbicides and can be applied in a variety of ways at various concentrations. In practice, the compounds herein defined are formulated into herbicidal compositions, by admixture, in herbicidally effective amounts, with the adjuvants and carriers normally employed for facilitating the dispersion of active ingredients for agricultural applications, recognizing the fact that the formulation and mode of application of a toxicant may affect the activity of the materials in a given application. Thus, these active herbicidal compounds may be formulated as granules of relatively large particle size, as wettable powders, as emulsifiable concentrates, as powdery dusts, as solutions or as

any of several other known types of formulations, depending upon the desired mode of application. Preferred formulations for pre-emergence herbicidal applications are wettable powders, emulsifiable concentrated and granules. These formulations may contain as little as about 0.5% to as much as about 95% or more by weight of active ingredient. A herbicidally effective amount depends upon the nature of the seeds or plants to be controlled and the rate of application varies from about 0.05 to approximately 25 pounds per acre, preferably from about 0.1 to 10 pounds per acre.

Wettable powders are in the form of finely divided particles which disperse readily in water or other dispersants. The wettable powder is ultimately applied to the soil either as a dry dust or as a dispersion in water or other liquid. Typical carriers for wettable powders include fuller's earth, kaolin clays, silicas and other readily wet organic or inorganic diluents. Wettable powders normally are prepared to contain about 5% to about 95% of the active ingredient and usually also contain a small amount of wetting, dispering, or emulsifying agent to facilitate wetting and dispersion.

Emulsifiable concentrates are homogeneous liquid compositions which are dispersible in water or other dispersant, and may consist entirely of the active compound with a liquid or solid emulsifying agent, or may also contain a liquid carrier, such as xylene, heavy aromatic naphthal, isophorone and other nonvolatile organic solvents. For herbicidal application, these concentrates are dispersed in water or other liquid carrier and normally applied as a spray to the area to be treated. The percentage by weight of the essential active ingredient may vary according to the manner in which the composition is to be applied, but in general comprises about 0.5% to 95% of active ingredient by weight of the herbicidal composition.

Granular formulations wherein the toxicant is carried on relatively coarse particles, are usually applied without dilution to the area in which suppression of vegetation is desired. Typical carriers for granular formulations include sand, fuller's earth, bentonite clays, vermiculite, perlite and other organic or inorganic materials which absorb or which may be coated with the toxicant. Granular formulations normally are prepared to contain about 5% to about 25% of active ingredients which may include surface-active agents such heavy aromatic naphthas, kerosene or other petroleum fractions, or vegetable oils; and/or stickers such as destrins, glue or synthetic resins.

Typical wetting, dispersing or emulsifying agents used in agricultural formulations include, for example, the alkyl and alkylaryl sulfonates and sulfates and their sodium salts; polyhydric alcohols; and other types of surface-active agents, many of which are available in commerce. The surface-active agent, when used, normally comprises from 0.1% to 15% by weight of the herbicidal composition.

Dusts, which are free-flowing admixtures of the active ingredient with finely divided solids such as talc, clays, flours and other organic and inorganic solids which act as dispersants and carriers for the toxicant, are useful formulations for soil-incorporating application.

Pastes, which are homogeneous suspensions of a finely divided solid toxicant in a liquid carrier such as water or oil, are employed for specific purposes. These formulations normally contain about 5% to about 95% of active ingredient by weight, and may also contain small amounts of a wetting, dispersing or emulsifying agent to facilitate dispersion. For application, the pastes are normally diluted and applied as a spray to the area to be affected.

Other useful formulations for herbicidal applications include simple solutions of the active ingredient in a dispersant in which it is completely soluble at the desired concentration, such as acetone, alkylated naphthalenes, xylene and other organic solvents. Pressurized sprays, typically aerosols, wherein the active ingredient is dispersed in finely-divided form as a result of vaporization of a low-boiling dispersant solvent carrier, such as the Freons, may also be used.

The phytoxic compositions of this invention are applied to the plants in the conventional manner. Thus, the dust and liquid compositions can be applied to the plant by the use of power-dusters, boom and hand sprayers and spray dusters. The compositions can also be applied from airplanes as a dust or a spray because they are effective in very low dosages. In order to modify or control growth of germinating seeds or emerging seedlings, as a typical example, the dust and liquid compositions are applied to the soil according to conventional methods and are distributed in the soil to a depth of at least ½ inch below the soil surface. It is not necessary that the phytotoxic compositions be admixed with the soil particles since these compositions can also be applied merely by spraying or sprinkling the surface of the soil. The phytotoxic compositions of this invention can also be applied by addition to irrigation water supplied to the field to be treated. This method of application permits the penetration of the compositions into the soil as the water is absorbed therein. Dust compositions, granular compositions or liquid formulations applied to the surface of the soil can be distributed below the surface by conventional means such as discing, dragging or mixing operations.

Emulsifiable Concentrate Formulations

| General Formula with Ranges | | Specific Formula | |
|---|---|---|---|
| Herbicidal compound | 5—55 | herbicidal compound | 54 |
| surfactant(s) | 5—25 | propriertary blend of oil soluble sulfonates and polyoxyethylene ethers | 10 |
| solvent(s) | 20—90 | polar solvent | 27 |
| | | petroleum hydrocarbon | 9 |
| | 100% | | 100% |

Wettable Powder Formulations

| | | | |
|---|---|---|---|
| herbicidal compound | 3—90 | herbicidal compound | 80 |
| wetting agent | 0.5—2 | sodium dialkyl naphthalene sulfonate | 0.5 |
| dispersing agent | 1—8 | sodium lignosulfonate | 7 |
| diluent(s) | 8.5—87 | attapulgite clay | 12.5 |
| | 100% | | 100% |

Extruded Granular Formulations

| | | | |
|---|---|---|---|
| herbicidal compound | 1—20 | herbicidal compound | 10 |
| binding agent | 0—10 | lignin sulfonate | 5 |
| diluent(s) | 70—99 | calcium carbonate | 85 |
| | 100% | | 100% |

Flowable Formulations

| | | | |
|---|---|---|---|
| herbicidal compound | 20—70 | herbicidal compound | 45 |
| surfactant(s) | 1—10 | polyoxyethylene ether | 5 |
| suspending agent(s) | 0.05—1 | attagel | 0.05 |
| antifreeze agent | 1—10 | propylene glycol | 10 |
| antimicrobial agent | 1—10 | BIT | 0.03 |
| antifoam agent | 0.1—1 | silicone defoamer | 0.02 |
| solvent | 7.95—77.85 | water | 39.9 |
| | 100% | | 100% |

The phytotoxic compositions of this invention can also contain other additaments, for example, fertilizers and other herbicides, pesticides and the like, used as adjuvant or in combination with any of the above-described adjuvants. Other phytotoxic compounds useful in combination with the above-described compounds include, for example, anilides such as 2-benzothiazole-2-yloxy-N-methyl acetanilide, 2-chloro-2',6'-dimethyl-N-(n-propylethyl) acetanilide, 2-chloro-2',6'-diethyl-N-(butoxymethyl) acetanilide; 2,4-dichlorophenoxyacetic acids, 2,4,5-trichlorophenoxyacetic acid, 2-methyl-4-chlorophenoxyacetic acid and the salts, esters and amides thereof; triazine derivatives, such as 2,4-bis(3-methoxypropylamino)-6-methylthio-s-triazine, 2-chloro-4-ethylamino-6-isopropylamino-s-triazine, and 2-ethylamino-4-isopropyl-amino-6-methyl-mercapto-s-triazine; urea derivatives, such as 3-(3,5-dichlorophenyl)-1,1-dimethylurea and 3-(p-chlorophenyl)-1,1-dimethylurea; and acetamides such as N,N-diallyl-α-chloroacetamide, and the like; benzoic acids such as 3-amino-2,5-dichlorobenzoic acid; thiocarbamates such as S-(1,1-dimethylbenzyl)-piperidene-1-carbothioate, 3-(4-chlorophenyl)-methyl diethylcarbothioate, ethyl-1-hexahydro-1,4-azepine-1-carbothioate, S-ethyl-hexahydro-1H-azepine-1-carbothioate, S-propyl N,N-dipropylthiocarbamate, S-ethyl, N,N-dipropylthiocarbamate, S-ethyl cyclohexylethylthiocarbamate and the like; anilines such as 4-(methylsulfonyl)-2,6-dinitro-N,N-substituted aniline, 4-trifluoromethyl-2,6-dinitro-N,N-di-n-propyl aniline, 4-trifluoromethyl-2,6-dinitro-N-ethyl-N-butyl aniline, 2-[4-(2,4-dichlorophenoxy)phenoxyl]-propanoic acid, 2-[1-(ethoxyimino)butyl]-5-[2-ethylthio)propyl]-3-hydroxy-2-cyclohexene-1-one, (±)-butyl-2[4-[(5-trifluoromethyl)-2-pyridinyl)oxy]-phenoxy]propanate, sodium 5-[2-chloro-4-(trifluoromethyl)phenoxy]-2-nitrobenzoate, 3-isopropyl-1H-2,1,3-benzothiadiazine-4(3H)-one-2,2-dioxide, and 4-amino-6-tert-butyl-3(methylthio)-as-triazin-5(4H)-one or 4-amino-6-(1,1-dimethylethyl)-3-(methylthio)-1,2,4-triazin-5(4H)-one and S-(O-O-diisopropyl)-benzene sulfonamide. Fertilizers useful in combination with the active ingredients include, for example, ammonium nitrate, urea and superphosphate. Other useful additaments include materials in which plant organisms take root and grow such as compost, manure, humus, sand, and the like.

**Claims**

1. A compound of the formula

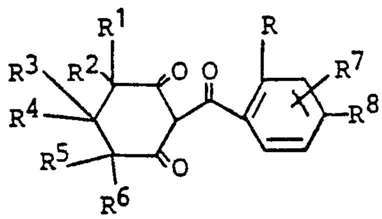

wherein

R is $C_1$—$C_4$ alkyl optionally substituted with halogen;

$R^1$ is hydrogen or $C_1$—$C_4$ alkyl;

$R^2$ is hydrogen, $C_1$—$C_4$ alkyl or

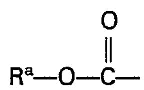

wherein $R^a$ is $C_1$—$C_4$ alkyl; or

$R^1$ and $R^2$ together are alkylene having 3 to 6 carbon atoms;

$R^3$ is hydrogen or $C_1$—$C_4$ alkyl;

$R^4$ is hydrogen or $C_1$—$C_4$ alkyl;

$R^5$ is hydrogen or $C_1$—$C_4$ alkyl;

$R^6$ is hydrogen or $C_1$—$C_4$ alkyl; and

$R^7$ and $R^8$ independently are (1) hydrogen; (2) halogen; (3) $C_1$—$C_4$ alkly; (4) $C_1$—$C_4$ alkoxy; (5) $OCF_3$; (6) cyano; (7) nitro; (8) $C_1$—$C_4$ haloalkyl; (9) $R^bSO_n$— wherein n is the integer 0, 1 or 2; and $R^b$ is (a) $C_1$—$C_4$ alkyl; (b) $C_1$—$C_4$ alkyl substituted with halogen or cyano; (c) phenyl; or (d) benzyl; (10) —$NR^cR^d$ wherein $R^c$ and $R^d$ independently are hydrogen or $C_1$—$C_4$ alkyl; (11) $R^eC(O)$— wherein $R^e$ is $C_1$—$C_4$ alkyl or $C_1$—$C_4$ alkoxy; or (12) $SO_2NR^cR^d$ wherein $R^c$ and $R^d$ are as defined, with the proviso that $R^7$ is not attached to the 6-position.

2. The compounds of Claim 1 wherein R is methyl or $CF_3$; $R^1$ is hydrogen or methyl; $R^2$ is hydrogen or methyl; $R^3$ is hydrogen or methyl; $R^4$ is hydrogen or methyl; $R^5$ is hydrogen or methyl; $R^6$ is hydrogen or methyl; $R^7$ and $R^8$ independently are (1) hydrogen; (2) halogen; (3) $C_1$—$C_4$ alkyl; (4) $C_1$—$C_4$ alkoxy; (5) $OCF_3$; (6) cyano; (7) nitro; (8) $C_1$—$C_4$ haloalkyl; (9) $R^bSO_n$- wherein n is the integer 0, 1 or 2; and $R^b$ is (a) $C_1$—$C_4$ alkyl; (b) $C_1$—$C_4$ alkyl substituted with halogen or cyano; (c) phenyl; or (d) benzyl; (10) —$NR^cR^d$ wherein $R^c$ and $R^d$ independently are hydrogen or $C_1$—$C_4$ alkyl; (11) $R^eC(O)$—wherein $R^e$ is $C_1$—$C_4$ alkyl or $C_1$—$C_4$ alkoxy; or (12) —$SO_2NR^cR^d$ wherein $R^c$ and $R^d$ are as defined.

3. The compounds of Claim 2 wherein $R^7$ and $R^8$ are independently are hydrogen; chlorine, fluorine, bromine; methyl; methoxy; $OCF_3$; cyano; nitro; trifluoromethyl; $R^bSO_n$— wherein n is the integer 2 and $R^b$ is methyl, chloromethyl, trifluoromethyl, cyanomethyl, ethyl, or n-propyl; —$NR^cR^d$ wherein $R^c$ and $R^d$ independently are hydrogen or $C_1$—$C_4$ alkyl; $R^eC(O)$— where $R^e$ is $C_1$—$C_4$ alkyl or $C_1$—$C_4$ alkoxy or $SO_2NR^cR^d$ wherein $R^c$ and $R^d$ are as defined and $R^7$ is in the 3-position.

4. The compound of Claim 2 wherein $R^7$ is hydrogen and $R^8$ is hydrogen, chlorine, bromine, flourine, $CF_3$ or $R^bSO_2$ wherein $R^b$ is $C_1$—$C_4$ alkyl.

5. The compound of Claim 2 wherein R is methyl; $R^1$ is methyl; $R^2$ is methyl; $R^3$ is hydrogen; $R^4$ is hydrogen, $R^5$ is hydrogen; $R^6$ is hydrogen; $R^7$ is hydrogen; and $R^8$ is $CH_3SO_2$—.

6. The compound of Claim 2 wherein R is methyl; $R^1$ is methyl; $R^2$ is methyl; $R^3$ is hydrogen; $R^4$ is hydrogen, $R^5$ is methyl; $R^6$ is hydrogen; $R^7$ is hydrogen; and $R^8$ is $CH_3SO_2$—.

7. The compound of Claim 2 wherein R is methyl; $R^1$ is methyl; $R^2$ is methyl; $R^3$ is hydrogen; $R^4$ is hydrogen, $R^5$ is hydrogen; $R^6$ is hydrogen; $R^7$ is hydrogen; and $R^8$ is $C_2H_5SO_2$—.

8. The compound of Claim 2 wherein R is methyl; $R^1$ is hydrogen; $R^2$ is methyl; $R^3$ is hydrogen; $R^4$ is hydrogen, $R^5$ is hydrogen; $R^6$ is hydrogen; $R^7$ is hydrogen; and $R^8$ is $CH_3SO_2$—.

9. The compound of Claim 2 wherein R is methyl; $R^1$ is methyl; $R^2$ is methyl; $R^3$ is hydrogen; $R^4$ is hydrogen, $R^5$ is hydrogen; $R^6$ is hydrogen; $R^7$ is hydrogen; and $R^8$ is methylthio.

10. The compound of Claim 2 wherein R is methyl; $R^1$ is hydrogen; $R^2$ is hydrogen; $R^3$ is hydrogen; $R^4$ is hydrogen, $R^5$ is hydrogen; $R^6$ is hydrogen; $R^7$ is hydrogen; and $R^8$ is cyano.

11. The compound of Claim 2 wherein R is methyl; $R^1$ is hydrogen; $R^2$ is hydrogen; $R^3$ is hydrogen; $R^4$ is hydrogen, $R^5$ is hydrogen; $R^6$ is hydrogen; $R^7$ is hydrogen; and $R^8$ is $C_2H_5SO_2$—.

12. The compound of Claim 2 wherein R is $CF_3$; $R^1$ is methyl; $R^2$ is methyl; $R^3$ is hydrogen; $R^4$ is hydrogen, $R^5$ is methyl; $R^6$ is hydrogen; $R^7$ is hydrogen; and $R^8$ is bromine.

13. The compound of Claim 2 wherein R is methyl; $R^1$ is hydrogen; $R^2$ is hydrogen; $R^3$ is hydrogen; $R^4$ is hydrogen, $R^5$ is hydrogen; $R^6$ is hydrogen; $R^7$ is 3-cyano; and $R^8$ is hydrogen.

14. The compound of Claim 2 wherein R is $CF_3$; $R^1$ is methyl; $R^2$ is methyl; $R^3$ is hydrogen; $R^4$ is

hydrogen, $R^5$ is methyl; $R^6$ is hydrogen; $R^7$ is hydrogen; and $R^8$ is bromine.

15. The compound of Claim 2 wherein R is methyl; $R^1$ is methyl; $R^2$ is methyl; $R^3$ is hydrogen; $R^4$ is hydrogen, $R^5$ is hydrogen; $R^6$ is hydrogen; $R^7$ is 3-nitro; and $R^8$ is hydrogen.

16. The compounds of Claim 2 wherein $R^7$ is hydrogen.

17. The compounds of Claim 3 wherein $R^7$ is hydrogen.

18 The compound of Claim 1 wherein $R^1$ and $R^2$ are hydrogen or both methyl.

19 The compound of Claim 18 wherein $R^8$ is $—SO_2CH_3$, $—SO_2CH_2Cl$, $CF_3$, $—SO_2CH_3$, cyano, $—SO_2CH_2Cl$ or $—SO_2C_2H_5$.

20. The method of controlling undesirable vegetation comprising applying to the area where control is desired, an herbicidally effective amount of a compound from Claims 1—19.

21. An herbical composition comprising an herbicidally active 2-(2-substituted benzoyl)-1,3-cyclohexanedione and an inert carrier therefor wherein the 2-benzoyl substituent is $C_1—C_4$ alkyl optionally substituted with halogen.

22. The herbicidal composition of Claim 21 wherein the 2-(2-substituted benzoyl)-1,3-cyclohexanedione is a compound of Claims 1—19.

23. The method of controlling undesirable vegetation comprising applying to the area where control is desired, an herbicidal composition comprising an herbicidally active 2-(2-benzoyl)-1,3-cyclohexanedione and an inert carrier therefor wherein the 2-position of the benzoyl moiety is substituted with $C_1—C_4$ alkyl, optionally substituted with halogen.

24. The method of Claim 23 wherein the 2-(2-benzoyl)-1,3-cyclohexanedione has a $C_1—C_4$ alkylsulfonyl or $C_1—C_4$ haloalkylsulfonyl substitution on the phenyl ring.

25. The method of Claim 24 wherein said alkylsulfonyl or haloalkylsulfonyl substitution is at the 4-position of the phenyl ring.

26. The herbicidal composition of Claim 21 wherein the 2-(2-benzoyl)-1,3-cyclohexanedione has a $C_1—C_4$ alkylsulfonyl or $C_1—C_4$ haloalkylsulfonyl substitution on the phenyl ring.

27. The herbicidal composition of Claim 26 wherein said alkylsulfonyl or haloalkylsulfonyl substitution is at the 4-position of the phenyl ring.

28. The method of Claim 23 wherein the 2-(2-benzoyl)-1,3-cyclohexanedione has a $C_1—C_4$ haloalkyl substitution on the phenyl ring.

29. The method of Claim 28 wherein said haloalkyl substitution is at the 4-position on the phenyl ring.

30. The herbicidal composition of Claim 21 wherein the 2-(2-benzoyl)-1,3-cyclohexanedione has a $C_1—C_4$ haloalkyl substitution on the phenyl ring.

31. The herbicidal composition of Claim 30 wherein said haloalkyl substitution is at the 4-position of the phenyl ring.

32. The herbicidal composition of Claim 30 wherein said haloalkyl is $CF_3$.

33. The method of Claim 20 wherein $R^1$ and $R^2$ are hydrogen or both methyl.

34. The method of Claim 33 wherein $R^8$ is $—SO_2CH_3$, $—SO_2CH_2Cl$, $CF_3$, $—SO_2CH_3$, cyano, $—SO_2CH_2Cl$ or $—SO_2C_2H_5$.

35. The composition of matter of Claim 22 wherein $R^1$ and $R^2$ are hydrogen or both methyl.

36. The composition of matter of Claim 35 wherein $R^8$ is $—SO_2CH_3$, $—SO_2CH_2Cl$, $CF_3$, $—SO_2CH_3$, cyano, $—SO_2CH_2Cl$ or $—SO_2C_2H_5$.

37. A process for preparing a compound of the formula

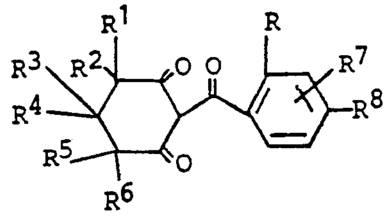

wherein

R is $C_1—C_4$ alkyl, optionally substituted with halogen;

$R^1$ is hydrogen or $C_1—C_4$ alkyl;

$R^2$ is hydrogen, $C_1—C_4$ alkyl or

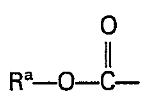

wherein $R^a$ is $C_1—C_4$ alkyl; or

$R^1$ and $R^2$ together are alkylene having 3 to 6 carbon atoms;

$R^3$ is hydrogen or $C_1—C_4$ alkyl;

$R^4$ is hydrogen or $C_1—C_4$ alkyl;

$R^5$ is hydrogen or $C_1—C_4$ alkyl;

$R^6$ is hydrogen or $C_1—C_4$ alkyl; and

$R^7$ and $R^8$ independently are (1) hydrogen; (2) halogen; (3) $C_1—C_4$ alkly; (4) $C_1—C_4$ alkoxy; (5) $OCF_3$; (6)

cyano; (7) nitro; (8) $C_1$—$C_4$ haloalkyl; (9) $R^bSO_n$- wherein n is the integer 0, 1 or 2; and $R^b$ is (a) $C_1$—$C_4$ alkyl; (b) $C_1$—$C_4$ alkyl substituted with halogen or cyano; (c) phenyl; or (d) benzyl; (10) —$NR^cR^d$ wherein $R^c$ and $R^d$ independently are hydrogen or $C_1$—$C_4$ alkyl; (11) $R^eC(O)$— wherein $R^e$ is $C_1$—$C_4$ alkyl or $C_1$—$C_4$ alkoxy; or (12) $SO_2NR^cR^d$ wherein $R^c$ and $R^d$ are as defined, with the proviso that $R^7$ is not attached to the 6-position comprising

(a) reacting a dione of the formula

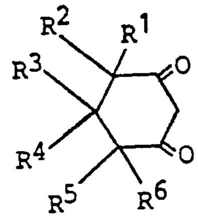

wherein $R^1$ through $R^6$ are as defined with a substituted benzoyl reactant of the formula

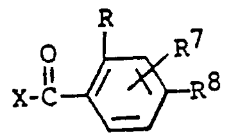

wherein R, $R^7$ and $R^8$ are as defined and X is halogen, $C_1$—$C_4$ alkyl—C(O)—O—, $C_1$—$C_4$ alkoxy—C(O)—O— or

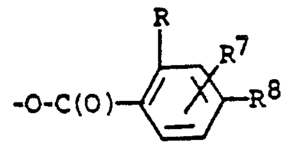

wherein R, $R^7$ and $R^8$ in this portion of the molecule are identical with those in the reactant shown above with at least a mole of a moderate base to form an enol ester of the formula

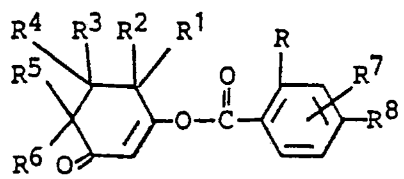

wherein R through $R^8$ are as defined and in step (2) reacting a mole of the enol ester intermediate with 1 to 4 moles of a moderate base, and from 0.01 mole to about 0.5 mole or higher of a cyanide source to form a compound of the formula

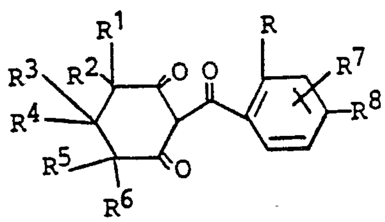

wherein $R^1$ through $R^8$ are as defined above.

38. The process of Claim 37 wherein X is halogen, the moderate base is tri-$C_1$—$C_6$ alkylamine, pyridine, alkali metal carbonate or alkali metal phosphate and the cyanide source alkali metal cyanide, cyanohydrins of methyl $C_1$—$C_4$ alkyl ketones, cyanohydrins of benzaldehyde or $C_2$—$C_5$ aliphatic aldehydes; cyanohydrins, zinc cyanide; tri(lower alkyl) silyl cyanides or hydrogen cyanide.

39. The process of Claim 38 wherein X is chlorine, the moderate base is tri-$C_1$—$C_6$ alkylamine, pyridine, sodium carbonate or sodium phosphate and the cyanide source is potassium cyanide, acetone cyanohydrin or hydrogen cyanide.

**Patentansprüche**

1. Verbindung der Formel

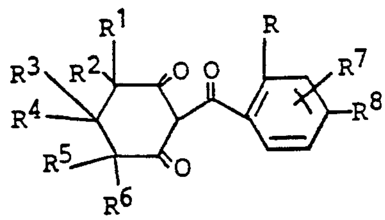

worin

R $C_1$—$C_4$-Alkyl, gegebenenfalls substituiert durch Halogen, bedeutet;
$R^1$ Wasserstoff oder $C_1$—$C_4$-Alkyl bedeutet;
$R^2$ Wasserstoff, $C_1$—$C_4$-Alkyl oder

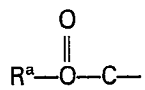

bedeutet, worin $R^a$ $C_1$—$C_4$-Alkyl bedeutet; oder worin
$R^1$ und $R^2$ zusammen Alkylen mit 3 bis 6 Kohlenstoffatomen bedeuten;
$R^3$ Wasserstoff oder $C_1$—$C_4$-Alkyl bedeutet;
$R^4$ Wasserstoff oder $C_1$—$C_4$-Alkyl bedeutet;
$R^5$ Wasserstoff oder $C_1$—$C_4$-Alkyl bedeutet;
$R^6$ Wasserstoff oder $C_1$—$C_4$-Alkyl bedeutet; und
$R^7$ und $R^8$ unabhängig (1) Wasserstoff; (2) Halogen; (3) $C_1$—$C_4$-Alkyl; (4) $C_1$—$C_4$-Alkoxy; (5) $OCF_3$; (6) Cyano; (7) Nitro; (8) $C_1$—$C_4$-Haloalkyl; (9) $R^bSO_n$—, worin n die ganze Zahl 0, 1 oder 2 bedeutet und $R^b$ (a) $C_1$—$C_4$-Alkyl, (b) $C_1$—$C_4$-Alkyl, substituiert mit Halogen oder Cyano, (c) Phenyl oder (d) Benzyl bedeutet; (10) —$NR^cR^d$, worin $R^c$ und $R^d$ unabhängig Wasserstoff oder $C_1$—$C_4$-Alkyl bedeuten; (11) $R^eC(O)$—, worin $R^e$ $C_1$—$C_4$-Alkyl oder $C_1$—$C_4$-Alkoxy bedeutet; oder (12) $SO_2NR^cR^d$, worin $R^c$ und $R^d$ die gegebenen Definitionen besitzen, bedeuten, mit der Maßgabe, daß $R^7$ nicht an die 6-Stellung gebunden ist.

2. Verbindungen nach Anspruch 1, dadurch gekennzeichnet, daß R Methyl oder $CF_3$ bedeutet; $R^1$ Wasserstoff oder Methyl bedeutet; $R^2$ Wasserstoff oder Methyl bedeutet; $R^3$ Wasserstoff oder Methyl bedeutet; $R^4$ Wasserstoff oder Methyl bedeutet; $R^5$ Wasserstoff oder Methyl bedeutet; $R^6$ Wasserstoff oder Methyl bedeutet; $R^7$ und $R^8$ unabhängig (1) Wasserstoff; (2) Halogen; (3) $C_1$—$C_4$-Alkyl; (4) $C_1$—$C_4$-Alkoxy; (5) $OCF_3$; (6) Cyano; (7) Nitro; (8) $C_1$—$C_4$-Haloalkyl; (9) $R^bSO_n$—, worin n die ganze Zahl 0, 1 oder 2 bedeutet und $R^b$ (a) $C_1$—$C_4$-Alkyl; (b) $C_1$—$C_4$-Alkyl, substituiert mit Halogen oder Cyano; (c) Phenyl oder (d) Benzyl bedeutet; (10) —$NR^cR^d$, worin $R^c$ und $R^d$ unabhängig Wasserstoff oder $C_1$—$C_4$-Alkyl bedeuten; (11) $R^eC(O)$—, worin $R^e$ $C_1$—$C_4$-Alkyl oder $C_1$—$C_4$-Alkoxy bedeutet; oder (12) $SO_2NR^cR^d$, worin $R^c$ und $R^d$ die gegebenen Definitionen besitzen, bedeuten.

3. Verbindungen nach Anspruch 2, dadurch gekennzeichnet, daß $R^7$ und $R^8$ unabhängig Wasserstoff, Chlor, Fluor, Brom, Methyl, Methoxy, $OCF_3$, Cyano, Nitro, Trifluormethyl, $R^bSO_n$—, worin n die ganze Zahl 2 ist und $R^b$ Methyl, Chlormethyl, Trifluormethyl, Cyanomethyl, Ethyl oder n-Propyl bedeutet, —$NR^cR^d$, worin $R^c$ und $R^d$ unabhängig Wasserstoff oder $C_1$—$C_4$-Alkyl bedeuten, $R^eC(O)$—, worin $R^e$ $C_1$—$C_4$-Alkyl oder $C_1$—$C_4$-Alkoxy bedeutet, oder $SO_2NR^cR^d$, worin $R^c$ und $R^d$ die gegebenen Definitionen besitzen, bedeuten und $R^7$ in der 3-Stellung vorhanden ist.

4. Verbindung nach Anspruch 2, dadurch gekennzeichnet, daß $R^7$ Wasserstoff bedeutet und $R^8$ Wasserstoff, Chlor, Brom, Fluor, $CF_3$ oder $R^bSO_2$ bedeutet, worin $R^b$ $C_1$—$C_4$-Alkyl bedeutet.

5. Verbindung nach Anspruch 2, dadurch gekennzeichnet, daß R Methyl; $R^1$ Methyl; $R^2$ Methyl; $R^3$ Wasserstoff; $R^4$ Wasserstoff; $R^5$ Wasserstoff; $R^6$ Wasserstoff; $R^7$ Wasserstoff und $R^8$ $CH_3SO_2$— bedeuten.

6. Verbindung nach Anspruch 2, dadurch gekennzeichnet, daß R Methyl; $R^1$ Methyl; $R^2$ Methyl; $R^3$ Wasserstoff; $R^4$ Wasserstoff; $R^5$ Methyl; $R^6$ Wasserstoff; $R^7$ Wasserstoff und $R^8$ $CH_3SO_2$— bedeuten.

7. Verbindung nach Anspruch 2, dadurch gekennzeichnet, daß R Methyl; $R^1$ Methyl; $R^2$ Methyl; $R^3$ Wasserstoff; $R^4$ Wasserstoff; $R^5$ Methyl; $R^6$ Wasserstoff; $R^7$ Wasserstoff und $R^8$ $C_2H_5SO_2$— bedeuten.

8. Verbindung nach Anspruch 2, dadurch gekennzeichnet, daß R Methyl; $R^1$ Wasserstoff; $R^2$ Wasserstoff; $R^3$ Wasserstoff; $R^4$ Wasserstoff; $R^5$ Wasserstoff; $R^6$ Wasserstoff; $R^7$ Wasserstoff und $R^8$ $CH_3SO_2$— bedeuten.

9. Verbindung nach Anspruch 2, dadurch gekennzeichnet, daß R Methyl; $R^1$ Methyl; $R^2$ Methyl; $R^3$ Wasserstoff; $R^4$ Wasserstoff; $R^5$ Wasserstoff; $R^6$ Wasserstoff; $R^7$ Wasserstoff und $R^8$ Methylthio bedeuten.

10. Verbindung nach Anspruch 2, dadurch gekennzeichnet, daß R Methyl; $R^1$ Wasserstoff; $R^2$ Wasserstoff; $R^3$ Wasserstoff; $R^4$ Wasserstoff; $R^5$ Wasserstoff; $R^6$ Wasserstoff; $R^7$ Wasserstoff und $R^8$ Cyano bedeuten.

11. Verbindung nach Anspruch 2, dadurch gekennzeichnet, daß R Methyl; $R^1$ Wasserstoff; $R^2$ Wasser-

stoff; $R^3$ Wasserstoff; $R^4$ Wasserstoff; $R^5$ Wasserstoff; $R^6$ Wasserstoff; $R^7$ Wasserstoff und $R^8$ $C_2H_5SO_2$— bedeuten.

12. Verbindung nach Anspruch 2, dadurch gekennzeichnet, daß R $CF_3$; $R^1$ Methyl; $R^2$ Methyl; $R^3$ Wasserstoff; $R^4$ Wasserstoff; $R^5$ Methyl; $R^6$ Wasserstoff; $R^7$ Wasserstoff und $R^8$ Brom bedeuten.

13. Verbindung nach Anspruch 2, dadurch gekennzeichnet, daß R Methyl; $R^1$ Wasserstoff; $R^2$ Wasserstoff; $R^3$ Wasserstoff; $R^4$ Wasserstoff; $R^5$ Wasserstoff; $R^6$ Wasserstoff; $R^7$ 3-Cyano und $R^8$ Wasserstoff bedeuten.

14. Verbindung nach Anspruch 2, dadurch gekennzeichnet, daß R $CF_3$; $R^1$ Methyl; $R^2$ Methyl; $R^3$ Wasserstoff; $R^4$ Wasserstoff; $R^5$ Methyl; $R^6$ Wasserstoff; $R^7$ Wasserstoff und $R^8$ Brom bedeuten.

15. Verbindung nach Anspruch 2, dadurch gekennzeichnet, daß R Methyl; $R^1$ Methyl; $R^2$ Methyl; $R^3$ Wasserstoff; $R^4$ Wasserstoff; $R^5$ Wasserstoff; $R^6$ Wasserstoff; $R^7$ 3-Nitro und $R^8$ Wasserstoff bedeuten.

16. Verbindung nach Anspruch 2, dadurch gekennzeichnet, daß $R^7$ Wasserstoff bedeutet.

17. Verbindung nach Anspruch 3, dadurch gekennzeichnet, daß $R^7$ Wasserstoff bedeutet.

18. Verbindung nach Anspruch 1, dadurch gekennzeichnet, daß $R^1$ und $R^2$ Wasserstoff oder beide Methyl bedeuten.

19. Verbindung nach Anspruch 18, dadurch gekennzeichnet, daß $R^8$ $—SO_2CO_3$, $—SO_2CH_2Cl$, $CF_3$, $—SO_2CH_3$, Cyano, $—SO_2CH_2Cl$ oder $—SO_2C_2H_5$ bedeutet.

20. Verfahren zur Kontrolle unerwünschter Vegetation, dadurch gekennzeichnet, daß man auf die Fläche, wo eine Kontrolle erfolgen soll, eine herbizid wirksame Menge einer Verbindung der Ansprüche 1 bis 19 anwendet.

21. Herbizides Mittel, dadurch gekennzeichnet, daß es ein herbizid aktives 2-(2-substituiertes-Benzoyl)-1,3-cyclohexandion und einen inerten Träger dafür enthält, wobei der 2-Benzoyl- Substituent $C_1$—$C_4$-Alkyl, gegebenenfalls substituiert mit Halogen, ist.

22. Herbizides Mittel nach Anspruch 21, dadurch gekennzeichnet, daß das 2-(2-substituierte-Benzoyl)-1,3-cyclohexandion eine Verbindung der Ansprüche 1 bis 19 ist.

23. Verfahren zur Kontrolle unerwünschter Vegetation, dadurch gekennzeichnet, daß man auf die Fläche, wo eine Kontrolle erfolgen soll, ein herbizides Mittel anwendet, welches ein herbizid aktives 2-(2-Benzoyl)-1,3-cyclohexandion und einen inerten Träger dafür enthält, wobei die 2-Stellung des Benzoyl-Molekülteils durch $C_1$—$C_4$-Alkyl, gegebenenfalls substituiert mit Halogen, substituiert ist.

24. Verfahren nach Anspruch 23, dadurch gekennzeichnet, daß das 2-(2-Benzoyl)-1,3-cyclohexandion eine $C_1$—$C_4$-Alkylsulfonyl- oder $C_1$—$C_4$-Haloalkylsulfonyl-Substitution an dem Phenylring aufweist.

25. Verfahren nach Anspruch 24, dadurch gekennzeichnet, daß die Alkylsulfonyl- oder Haloalkylsulfonyl-Substitution in der 4-Stellung des Phenylrings vorhanden ist.

26. Herbizides Mittel nach Anspruch 21, dadurch gekennzeichnet, daß das 2-(2-Benzoyl)-1,3-cyclohexandion eine $C_1$—$C_4$-Alkylsulfonyl- oder $C_1$—$C_4$-Haloalkylsulfonyl-Substitution am Phenylring aufweist.

27. Herbizides Mittel nach Anspruch 26, dadurch gekennzeichnet, daß die Alkylsulfonyl- oder Haloalkylsulfonyl-Substitution in der 4-Stellung des Phenylrings vorhanden ist.

28. Verfahren nach Anspruch 23, dadurch gekennzeichnet, daß das 2-(2-Benzoyl)-1,3-cyclohexandion eine $C_1$—$C_4$-Haloalkyl-Substitution am Phenylring aufweist.

29. Verfahren nach Anspruch 28, dadurch gekennzeichnet, daß die Haloalkyl-Substitution in der 4-Stellung des Phenylrings vorhanden ist.

30. Verfahren nach Anspruch 21, dadurch gekennzeichnet, daß das 2-(2-Benzoyl)-1,3-cyclohexandion eine $C_1$—$C_4$-Haloalkyl-Substitution am Phenylring aufweist.

31. Herbizides Mittel nach Anspruch 30, dadurch gekennzeichnet, daß die Haloalkyl-Substitution in der 4-Stellung des Phenylrings vorhanden ist.

32. Herbizides Mittel nach Anspruch 30, dadurch gekennzeichnet, daß das Haloalkyl $CF_3$ ist.

33. Verfahren nach Anspruch 20, dadurch gekennzeichnet, daß $R^1$ und $R^2$ Wasserstoff oder beide Methyl bedeuten.

34. Verfahren nach Anspruch 33, dadurch gekennzeichnet, daß $R^8$ $—SO_2CH_3$, $—SO_2CH_2Cl$, $CF_3$, $—SO_2CH_3$, Cyano, $—SO_2CH_2Cl$ oder $—SO_2C_2H_5$ bedeutet.

35. Mittel nach Anspruch 22, dadurch gekennzeichnet, daß $R^1$ und $R^2$ Wasserstoff oder beide Methyl bedeuten.

36. Mittel nach Anspruch 35, dadurch gekennzeichnet, daß $R^8$ $—SO_2CH_3$, $—SO_2CH_2Cl$, $CF_3$, $—SO_2CH_3$, Cyano, $—SO_2CH_2Cl$ oder $—SO_2C_2H_5$ bedeutet.

37. Verfahren zur Herstellung einer Verbindung der Formel

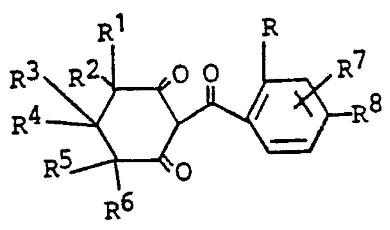

worin
R $C_1$—$C_4$-Alkyl, gegebenenfalls substituiert mit Halogen, bedeutet;
$R^1$ Wasserstoff oder $C_1$—$C_4$-Alkyl bedeutet;
$R^2$ Wasserstoff, $C_1$—$C_4$-Alkyl oder

O
‖
$R^a$—O—C—

bedeutet, worin $R^a$ $C_1$—$C_4$-Alkyl bedeutet; oder
$R^1$ und $R^2$ zusammen Alkylen mit 3 bis 6 Kohlenstoffatomen bedeuten;
$R^3$ Wasserstoff oder $C_1$—$C_4$-Alkyl bedeutet;
$R^4$ Wasserstoff oder $C_1$—$C_4$-Alkyl bedeutet;
$R^5$ Wasserstoff oder $C_1$—$C_4$-Alkyl bedeutet;
$R^6$ Wasserstoff oder $C_1$—$C_4$-Alkyl bedeutet; und
$R^7$ und $R^8$ unabhängig (1) Wasserstoff; (2) Halogen; (3) $C_1$—$C_4$-Alkyl; (4) $C_1$—$C_4$-Alkoxy; (5) $OCF_3$; (6) Cyano; (7) Nitro; (8) $C_1$—$C_4$-Haloalkyl; (9) $R^bSO_n$—, worin n die ganze Zahl 0, 1 oder 2 ist und $R^b$ (a) $C_1$—$C_4$-Alkyl; (b) $C_1$—$C_4$-Alkyl, substituiert mit Halogen oder Cyano; (c) Phenyl oder (d) Benzyl bedeutet; (10) —$NR^cR^d$, worin $R^c$ und $R^d$ unabhängig Wasserstoff oder $C_1$—$C_4$-Alkyl bedeuten; (11) $R^eC(O)$—, worin $R^e$ $C_1$—$C_4$-Alkyl oder $C_1$—$C_4$-Alkoxy bedeutet; oder (12) $SO_2NR^cR^d$, worin $R^c$ und $R^d$ die gegebenen Definitionen besitzen, bedeuten, mit der Maßgabe, daß $R^7$ nicht an die 6-Stellung gebunden ist,
dadurch gekennzeichnet, daß man
(a) ein Dion der Formel

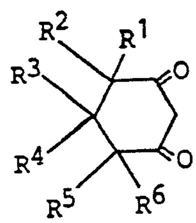

worin $R^1$ bis $R^6$ die gegebenen Definitionen besitzen, mit einem substituierten Benzoyl-Reaktionsteilnehmer der Formel

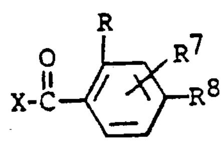

worin R, $R^7$ und $R^8$ die gegebenen Definitionen besitzen und X Halogen, $C_1$—$C_4$-Alkyl-C(O)—O—, $C_1$—$C_4$-Alkoxy—C(O)—O— oder

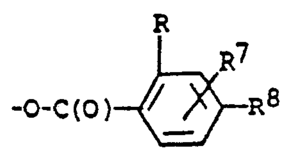

worin R, $R^7$ und $R^8$ in diesem Teil des Moleküls identisch sind mit denen in dem oben gezeigten Reaktionsteilnehmer, bedeutet, mit mindestens einem Mol einer mäßigen Base unter Bildung eines Enolesters der Formel

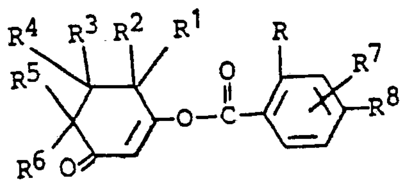

umsetzt, worin R bis $R^8$ die gegebenen Definitionen besitzen, und bei der Stufe (2) ein Mol des Enolester-Zwischenprodukts mit 1 bis 4 Mol einer milden Base und mit 0,01 bis etwa 0,5 Mol oder mehr einer Cyanidquelle unter Bildung einer Verbindung der Formel

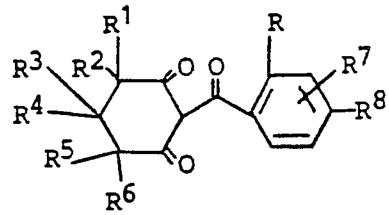

umsetzt, worin $R^1$ bis $R^8$ die oben gegebenen Definitionen besitzen.

38. Verfahren nach Anspruch 37, dadurchh gekennzeichnet, daß X Halogen bedeutet, die milde Base ein Tri-$C_1$—$C_6$-alkylamin, Pyridin, ein Alkalimetallcarbonat oder Alkalimetallphosphat ist und die Quelle für Cyanid ein Alkalimetallcyanid, Cyanohydrine von Methyl-$C_1$—$C_4$-alkylketonen, Cyanohydrinen von Benzaldehyd oder $C_2$—$C_5$-aliphatischen-Aldehyden, Cyanohydrinen, Zinkcyanid, Tri(niedrigalkyl)silylcyaniden oder Cyanwasserstoff ist.

39. Verfahren nach Anspruch 38, dadurch gekennzeichnet, daß X Chlor bedeutet, die milde Base Tri-$C_1$—$C_6$-alkylamin, Pyridin, Natriumcarbonat oder Natriumphosphat ist und die Cyanidquelle Kaliumcyanid, Acetoncyanohydrin oder Cyanwasserstoff ist.

## Revendications

1. Un dérivé de formule:

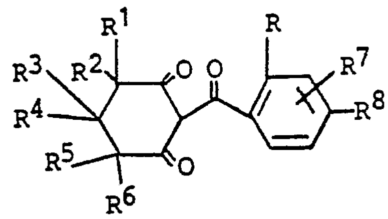

dans laquelle:

R représente un radical alkyle en $C_1$ à $C_4$, éventuellement substitué par un halogène;

$R^1$ représente un atome d'hydrogène ou un radical alkyle en $C_1$ à $C_4$,

$R^2$ représente un atome d'hydrogène, un radical alkyle en $C_1$ à $C_4$, ou:

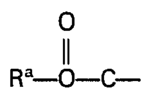

dans laquelle $R^a$ représente un radical alkyle en $C_1$ à $C_4$; ou

$R^1$ et $R^2$ représentent ensemble un radical alkylène renfermant de 3 à 6 atomes de carbone;

$R^3$ représente un atome d'hydrogène ou un radical alkyle en $C_1$ à $C_4$;

$R^4$ représente un atome d'hydrogène ou un radical alkyle en $C_1$ à $C_4$;

$R^5$ représente un atome d'hydrogène ou un radical alkyle en $C_1$ à $C_4$;

$R^6$ représente un atome d'hydrogène ou un radical alkyle en $C_1$ à $C_4$;

$R^7$ et $R^8$ représentent indépendamment: (1) un atome d'hydrogène; (2) un atome d'halogène; (3) un radical alkyle en $C_1$ à $C_4$; (4) un radical alkoxy en $C_1$ à $C_4$; (5) $OCF_3$; (6) cyano; (7) nitro; (8) un radical haloalkyle en $C_1$ à $C_4$; (9) $R^bSO_n$— dans lequel: n représente un nombre entier égal à 0, 1 ou 2; et $R^b$ représente: (a) un radical alkyle en $C_1$ à $C_4$; (b) alkyle en $C_1$ à $C_4$ substitué par un atome d'halogène ou un groupe cyano; (c) le radical phényle; ou (d) le radical benzyle; (10) —$NR^cR^d$ dans lequel: $R^c$ et $R^d$ représentent indépendamment un atome d'hydrogène ou un radical alkyle en $C_1$ à $C_4$; (11) $R^eC(O)$— dans lequel: $R^e$ représente un radical alkyle en $C_1$ à $C_4$ ou alkoxy en $C_1$ à $C_4$; ou (12) —$SO_2NR^cR^d$ dans lequel: $R^c$ et $R^d$ sont tels que définis ci-dessus, sous réserve que $R^7$ ne soit pas attaché en position 6.

2. Les dérivés selon la revendication 1, dans lequel R représente le radical méthyle ou $CF_3$; $R^1$ représente un atome d'hydrogène ou le radical méthyle; $R^2$ représente un atome d'hydrogène ou le radical méthyle; $R^3$ représente un atome d'hydrogène ou le radical méthyle; $R^4$ représente un atome d'hydrogène ou le radical méthyle; $R^5$ représente un atome d'hydrogène ou le radical méthyle; $R^6$ représente un atome d'hydrogène ou le radical méthyle; $R^7$ et $R^8$ représentent indépendamment (1) un atome d'hydrogène; (2) un atome d'halogène; (3) un radical alkyle en $C_1$ à $C_4$; (4) alkoxy en $C_1$ à $C_4$; (5) $OCF_3$; (6) cyano; (7) nitro; (8) un radical haloalkyle en $C_1$ à $C_4$; (9) $R^bSO_n$— dans lequel n est égal à 0, 1 ou 2 et $R^b$ représente (a) un radical alkyle en $C_1$ à $C_4$; (b) un radical alkyle en $C_1$ à $C_4$ substitué par un atome d'halogène ou un groupe cyano; (c) phényle ou (d) benzyle; (10) —$NR^cR^d$ dans lequel $R^c$ et $R^d$ représentent indépendamment un atome d'hydrogène ou un radical alkyle en $C_1$ à $C_4$; (11) $R^eC(O)$— dans lequel $R^e$ représente un radical alkyle en $C_1$ à $C_4$ ou alkoxy en $C_1$ à $C_4$; ou (12) $SO_2NR^cR^d$ dans lequel $R^c$ et $R^d$ sont tels que définis ci-dessus.

3. Les dérivés selon la revendication 2, dans lesquels $R^7$ et $R^8$ représentent indépendamment un atome d'hydrogène, de chlore, de fluor, de brome, un radical méthyle ou méthoxy, $OCF_3$, cyano, nitro, trifluorométhyle, $R^bSO_n$— dans lequel n représente un nombre entier égal à 2 et $R^b$ représente le radical méthyle, chlorométhyle, trifluorométhyle, cyanométhyle, éthyle ou n-propyle, —$NR^cR^d$ dans lequel $R^c$ et $R^d$ représentent indépendamment un atome d'hydrogène ou un radical alkyle en $C_1$ à $C_4$, $R^eC(O)$— dans lequel $R^e$ représente un radical alkyle en $C_1$ à $C_4$ ou alkoxy en $C_1$ à $C_4$, ou $SO_1NR^cR^d$ dans lequel $R^c$ et $R^d$ sont tels que définis et $R^7$ se trouve en position 3.

4. Le dérivé selon la revendication 2, dans lequel $R^7$ représente un atome d'hydrogène et $R^8$ représente un atome d'hydrogène, de chlore, de brome, de fluor, $CF_3$ ou $R^bSO_2$ dans lequel $R^b$ représente un radical alkyle en $C_1$ à $C_4$.

5. Le dérivé selon la revendication 2, dans lequel R représente le radical méthyle, $R^1$ représente le radical méthyle, $R^2$ représente le radical méthyle, $R^3$ représente un atome d'hydrogène, $R^4$ représente un atome d'hydrogène, $R^5$ représente un atome d'hydrogène, $R^6$ représente un atome d'hydrogène, $R^7$ représente un atome d'hydrogène et $R^8$ représente le radical $CH_3SO_2$—.

6. Le dérivé selon la revendication 2, dans lequel R représente le radical méthyle, $R^1$ représente le radical méthyle, $R^2$ représente le radical méthyle, $R^3$ représente un atome d'hydrogène, $R^4$ représente un atome d'hydrogène, $R^5$ représente le radical méthyle, $R^6$ représente un atome d'hydrogène, $R^7$ représente un atome d'hydrogène et $R^8$ représente le radical $CH_3SO_2$—.

7. Le dérivé selon la revendication 2, dans lequel R représente le radical méthyle, $R^1$ représente le radical méthyle, $R^2$ représente le radical methyle, $R^3$ représente un atome d'hydrogène, $R^4$ représente un atome d'hydrogène, $R^5$ représente un atome d'hydrogène, $R^6$ représente un atome d'hydrogène, $R^7$ représente un atome d'hydrogène et $R^8$ représente le radical $C_2H_5SO_2$—.

8. Le dérivé selon la revendication 2, dans lequel R représente le radical méthyle, $R^1$ représente un atome d'hydrogène, $R^2$ représente un atome d'hydrogène, $R^3$ représente un atome d'hydrogène, $R^4$ représente un atome d'hydrogène, $R^5$ représente un atome d'hydrogène, $R^6$ représente un atome d'hydrogène, $R^7$ représente un atome d'hydrogène et $R^8$ représente le radical $CH_3SO_2$—.

9. Le dérivé selon la revendication 2, dans lequel R représente le radical méthyle, $R^1$ représente le radical méthyle, $R^2$ représente le radical méthyle, $R^3$ représente un atome d'hydrogène, $R^4$ représente un atome d'hydrogène, $R^5$ représente un atome d'hydrogène, $R^6$ représente un atome d'hydrogène, $R^7$ représente un atome d'hydrogène et $R^8$ représente le radical méthylthio.

10. Le dérivé selon la revendication 2, dans lequel R représente le radical méthyle, $R^1$ représente un atome d'hydrogène, $R^2$ représente un atome d'hydrogène, $R^3$ représente un atome d'hydrogène, $R^4$ représente un atome d'hydrogène, $R^5$ représente un atome d'hydrogène, $R^6$ représente un atome d'hydrogène, $R^7$ représente un atome d'hydrogène et $R^8$ représente le groupe cyano.

11. Le dérivé selon la revendication 2, dans lequel R représente le radical méthyle, $R^1$ représente un atome d'hydrogène, $R^2$ représente un atome d'hydrogène, $R^3$ représente un atome d'hydrogène, $R^4$ représente un atome d'hydrogène, $R^5$ représente un atome d'hydrogène, $R^6$ représente un atome d'hydrogène, $R^7$ représente un atome d'hydrogène et $R^8$ représente le radical $C_2H_5SO_2$—.

12. Le dérivé selon la revendication 2, dans lequel R représente le $CF_3$, $R^1$ représente le radical méthyle, $R^2$ représente le radical méthyle, $R^3$ représente un atome d'hydrogène, $R^4$ représente un atome d'hydrogène, $R^5$ représente le radical méthyle, $R^6$ représente un atome d'hydrogène, $R^7$ représente un atome d'hydrogène et $R^8$ représente un atome de brome.

13. Le dérivé selon la revendication 2, dans lequel R représente le radical méthyle, $R^1$ représente un atome d'hydrogène, $R^2$ représente un atome d'hydrogène, $R^3$ représente un atome d'hydrogène, $R^4$ représente un atome d'hydrogène, $R^5$ représente un atome d'hydrogène, $R^6$ représente un atome d'hydrogène, $R^7$ représente le 3-cyano, et $R^8$ représente un atome d'hydrogène.

14. Le dérivé selon la revendication 2, dans lequel R représente le $CF_3$, $R^1$ représente le radical méthyle, $R^2$ représente le radical méthyle, $R^3$ représente un atome d'hydrogène, $R^4$ représente un atome d'hydrogène, $R^5$ représente le radical méthyle, $R^6$ représente un atome d'hydrogène, $R^7$ représente un atome d'hydrogène, et $R^8$ représente un atome de brome.

15. Le dérivé selon la revendication 2, dans lequel R représente le radical méthyle, $R^1$ représente le radical méthyle, $R^2$ représente le radical méthyle, $R^3$ représente un atome d'hydrogène, $R^4$ représente un atome d'hydrogène, $R^5$ représente un atome d'hydrogène, $R^6$ représente un atome d'hydrogène, $R^7$ représente le 3-nitro, et $R^8$ représente un atome d'hydrogène.

16. Les dérivés selon la revendication 2, dans lesquels $R^7$ représente un atome d'hydrogène.

17. Les dérivés selon la revendication 3, dans lesquels $R^6$ représente un atome d'hydrogène.

18. Le dérivé selon la revendication 1, dans lequel $R^1$ et $R^2$ représentent un atome d'hydrogène, ou représentent tous les deux le radical méthyle.

19. Le dérivé selon la revendication 18, dans lequel $R^8$ représente —$SO_2CH_3$, —$SO_2CH_2Cl$, $CF_3$, —$SO_2CH_3$, cyano, —$SO_2CH_2Cl$ ou —$SO_2C_2H_5$.

20. Le procédé de contrôle d'une végétation indésirable consistant à appliquer à la zone que l'on désire contrôler une quantité efficace en tant qu'herbicide d'un dérivé selon les revendications 1 à 19.

21. Une composition herbicide comprenant une 2-(2-benzoyl-substitué)-1,3-cyclohexanedione active en tant qu'herbicide et un support inerte de celle-ci, dans laquelle le substituant 2-benzoyle est un radical alkyle en $C_1$ à $C_4$, éventuellement substitué par un halogène.

22. La composition herbicide selon la revendication 21, dans laquelle la 2-(2-benzoyl-substitué)-1,3-cyclohexanedione est un dérivé selon les revendications 1 à 19.

23. Le procédé de contrôle d'une végétation indésirable consistant à appliquer à la zone que l'on désire contrôler une composition herbicide comprenant une 2-(2-benzoyl)-1,3-cyclohexanedione active en tant qu'herbicide et un support inerte de celle-ci, dans laquelle la position 2 du radical benzoyle est substituée par un radical alkyle en $C_1$ à $C_4$, éventuellement substitué par un halogène.

24. Le procédé selon la revendication 23, dans lequel la 2-(2-benzoyl)-1,3-cyclohexanedione comporte un radical alkylsulfonyle en $C_1$ à $C_4$ et haloalkylsulfonyle en $C_1$ à $C_4$ comme substitution sur le cycle du phényle.

25. Le procédé selon la revendication 24, dans lequel cette substitution par un radical alkylsulfonyle ou haloalkylsulfonyle se trouve en position 4 du cycle du phényle.

26. La composition herbicide selon la revendication 21, dans lequel la 2-(2-benzoyl)-1,3-cyclohexanedione présente une substitution par un radical alkylsulfonyle en $C_1$ à $C_4$ ou haloalkylsulfonyle en $C_1$ à $C_4$ sur le cycle du phényle.

27. La composition herbicide selon la revendication 26, dans laquelle cette substitution par un radical alkylsulfonyle ou haloalkylsulfonyle se trouve en position 4 du cycle du phényle.

28. Le procédé selon la revendication 23, dans lequel la 2-(2-benzoyl)-1,3-cyclohexanedione présente une substitution par un radical haloalkyle en $C_1$ à $C_4$ sur le cycle du phényle.

29. Le procédé selon la revendication 28, dans lequel cette substitution par un radical haloalkyle se trouve en position 4 sur le cycle du phényle.

30. La composition herbicide selon la revendication 21, dans laquelle la 2-(2-(benzoyl)-1,3-cyclohexanedione présente une substitution par un radical haloalkyle en $C_1$ à $C_4$ sur le cycle du phényle.

31. La composition herbicide selon la revendication 30, dans laquelle cette substitution par un radical haloalkyle se trouve en position 4 du cycle du phényle.

32. La composition herbicide selon la revendication 30, dans laquelle ce radical haloalkyle est le $CF_3$.

33. Le procédé selon la revendication 20, dans lequel $R^1$ et $R^2$ représentent un atome d'hydrogène ou représentent tous les deux le radical méthyle.

34. Le procédé selon la revendication 33, dans lequel $R^8$ représente $—SO_2CH_3$, $—SO_2CH_2Cl$, $CF_3$, $—SO_2CH_3$, cyano, $—SO_2CH_2Cl$ ou $—SO_2C_2H_5$.

35. La composition de produit selon la revendication 22, dans laquelle $R^1$ et $R^2$ représentent un atome d'hydrogène ou représentent tous les deux le radical méthyle.

36. La composition de produit selon la revendication 35, dans laquelle $R^8$ représente $—SO_2CH_3$, $—SO_2CH_2Cl$, $CF_3$, $—SO_2CH_3$, cyano, $—SO_2CHCl$ ou $—SO_2C_2H_5$.

37. Un procédé de préparation d'un dérivé de formule:

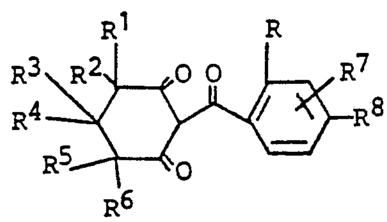

dans laquelle:

R représente un radical alkyle en $C_1$ à $C_4$, éventuellement substitué par un atome d'halogène;

$R^1$ représente un atome d'hydrogène ou un radical alkyle en $C_1$ à $C_4$,

$R^2$ représente un atome d'hydrogène, un radical alkyle en $C_1$ à $C_4$, ou:

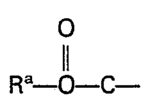

dans laquelle $R^a$ représente un radical alkyle en $C_1$ à $C_4$; ou

$R^1$ et $R^2$ représentent ensemble un radical alkylène renfermant de 3 à 6 atomes de carbone;

$R^3$ représente un atome d'hydrogène ou un radical alkyle en $C_1$ à $C_4$;

$R^4$ représente un atome d'hydrogène ou un radical alkyle en $C_1$ à $C_4$;

$R^5$ représente un atome d'hydrogène ou un radical alkyle en $C_1$ à $C_4$;

$R^6$ représente un atome d'hydrogène ou un radical alkyle en $C_1$ à $C_4$;

$R^7$ et $R^8$ représentent indépendamment: (1) un atome d'hydrogène; (2) un atome d'halogène; (3) un radical alkyle en $C_1$ à $C_4$; (4) un radical alkoxy en $C_1$ à $C_4$; (5) $OCF_3$; (6) cyano; (7) nitro; (8) un radical haloalkyle en $C_1$ à $C_4$; (9) $R^bSO_n—$ dans lequel: n représente un nombre entier égal à 0, 1 ou 2; et $R^b$ représente: (a) un radical alkyle en $C_1$ à $C_4$; (b) alkyle en $C_1$ à $C_4$ substitué par un atome d'halogène ou un groupe cyano; (c) le radical phényle; ou (d) le radical benzyle; (10) $—NR^cR^d$ dans lequel: $R^c$ et $R^d$ représentent indépendamment un atome d'hydrogène ou un radical alkyle en $C_1$ à $C_4$; (11) $R^eC(O)—$ dans lequel: $R^e$ représente un radical alkyle en $C_1$ à $C_4$ ou alkoxy en $C_1$ à $C_4$; ou (12) $—SO_2NR^cR^d$ dans lequel: $R^c$ et

$R^d$ sont tels que définis ci-dessus, sous réserve que $R^7$ ne soit pas attaché en position 6; comprenant les étapes suivantes:

(a) on fait réagir une dione de formule:

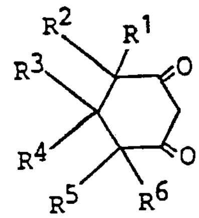

dans laquelle $R^1$ à $R^6$ sont tels que définis ci-dessus, avec un réactif benzylique substitué de formule:

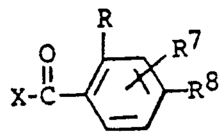

dans laquelle: R, $R^7$ et $R^8$ sont tels que définis ci-dessus; et X représente un atome d'halogène, un radical alkyl-C(O)—O— en $C_1$ à $C_4$, alkoxy-C(O)—O— en $C_1$ à $C_4$; ou

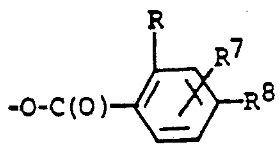

dans laquelle R, $R^7$ et $R^8$ dans cette portion de la moleculaire sont identiques à ceux du réactif représenté ci-dessus, avec au moins une mole d'une base modérée, pour former un ester-énol de formule:

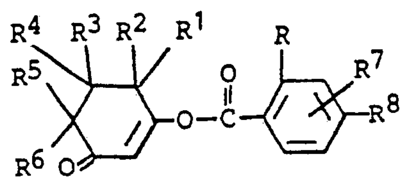

dans laquelle R à $R^8$ sont tels que définis ci-dessus; et dans l'étape (2) à faire réagir une mole de l'ester-énol intermédiaire avec 1 à 4 moles d'une base modérée et de 0,01 mole à environ 0,5 mole ou plus d'une source de cyanure pour former un dérivé de formule:

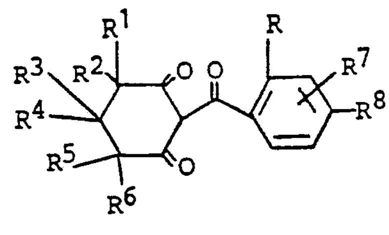

dans laquelle $R^1$ à $R^8$ sont tels que définis ci-dessus.

38. Le procédé selon la revendication 37, dans lequel X représente un atome d'halogène, la base modérée est une trialkylamine en $C_1$ à $C_6$, la pyridine, un carbonate de métal alcalin ou un phosphate de métal alcalin, et la source de cyanure est un cyanure de métal alcalin, des cyanhydrines de méthylalkylcétones en $C_1$ à $C_4$, des cyanhydrides de benzaldéhyde ou des cyanhydrines d'aldéhyde aliphatique en $C_2$ à $C_5$, du cyanure de zinc, des cyanures de trialkylsilyle inférieur ou de l'acide cyanhydrique gazeux.

39. Le procédé selon la revendication 38, dans lequel X représente un atome de chlore, la base modérée est une trialkylamine en $C_1$ à $C_6$, la pyridine, le carbonate de sodium ou le phosphate de sodium et la source de cyanure est le cyanure de potassium, l'acétone cyanhydrine ou l'acide cyanhydrique gazeux.